(19) Europäisches Patentamt
European Patent Office
Office européen des brevets

(11) EP 0 659 749 B1

(12) **EUROPÄISCHE PATENTSCHRIFT**

(45) Veröffentlichungstag und Bekanntmachung des Hinweises auf die Patenterteilung:
**20.09.2000 Patentblatt 2000/38**

(51) Int. Cl.$^7$: **C07D 279/20**, C07C 211/55, C07C 211/58, C08K 5/46

(21) Anmeldenummer: **94810727.1**

(22) Anmeldetag: **14.12.1994**

(54) **Mischungen kernalkylierter aromatischer Amine und Phenothiazine**

Mixtures of ringalkylated aromated amines and phenothiazines

Mélanges d'amines aromatiques alkylées sur le noyau et de phénothiazines

(84) Benannte Vertragsstaaten:
**BE DE ES FR GB IT NL**

(30) Priorität: **23.12.1993 CH 384593**

(43) Veröffentlichungstag der Anmeldung:
**28.06.1995 Patentblatt 1995/26**

(73) Patentinhaber:
**Ciba Specialty Chemicals Holding Inc.
4057 Basel (CH)**

(72) Erfinder:
- **Evans, Dr. Samuel
CH-1723 Marly (CH)**
- **Allenbach, Stephan
CH-3186 Düdingen (CH)**

(56) Entgegenhaltungen:
**EP-A- 0 149 422**
**EP-A- 0 275 910**
**EP-A- 0 417 036**
**EP-A- 0 475 904**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 659 749 B1

Printed by Xerox (UK) Business Services
2.16.7 (HRS)/3.6

## Beschreibung

Die Erfindung betrifft neue, als Antioxidantien geeignete, alkylierte Diphenylamine und Phenothiazine enthaltende Reaktionsprodukte, sie enthaltende Zusammensetzungen, ihre Verwendung und ein Verfahren zu ihrer Herstellung.

Flüssige Mischungen aminischer Antioxidantien werden seit geraumer Zeit, vornehmlich in Schmierölen für Verbrennungsmotoren, eingesetzt.
Insbesondere beschreiben EP-A-0,149,422 ein Gemisch von unsubstituiertem und verschiedentlich mit Alkyl substituiertem Diphenylamin und EP-A-0,475,904 ein aus der Umsetzung solcher Gemische mit Schwefel resultierendes, Phenothiazin(e) enthaltendes Stoffgemisch.

In EP-A-0,275,910 wird ferner die Herstellung von Mischungen beschrieben, die aus mit phenolischen Gruppen substituierten Diphenylamin- und Phenothiazinverbindungen bestehen.

Überraschenderweise wurde nun gefunden, daß man nach dem im folgenden beschriebenen Verfahren ein flüssiges Additivgemisch erhält, welches vor allem in Schmiermitteln einsetzbar ist und dort besonders zur Verhütung von Schlammablagerungen (Deposit Control) geeignet ist.

Die Erfindung betrifft daher das
Reaktionsprodukt, erhältlich aus der Umsetzung eines Olefins der Formel

$$R_1R_2C{=}CHR_3 \qquad (I)$$

mit
einer Mischung von Verbindungen der Formeln II und III

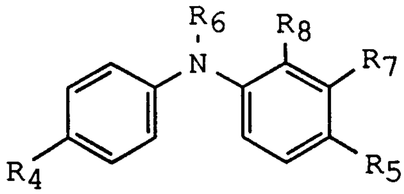 (II)

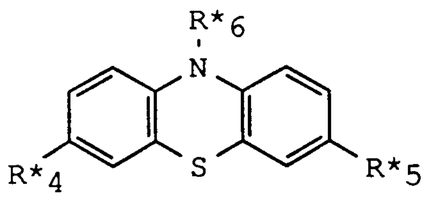 (III)

in Gegenwart eines sauren Katalysators,

wobei $R_1$ Wasserstoff, $C_1$-$C_{25}$-Alkyl oder Phenyl,
$R_2$ $C_1$-$C_{25}$-Alkyl, Benzyl oder Phenyl,
$R_3$ Wasserstoff oder $C_1$-$C_{12}$-Alkyl,
$R_4$, $R^*_4$, $R_5$ und $R^*_5$ unabhängig voneinander Wasserstoff, $C_1$-$C_{25}$-Alkyl, $C_5$-$C_{12}$-Cycloalkyl, Benzyl, $\alpha$-Methylbenzyl oder $\alpha,\alpha$-Dimethylbenzyl, und
$R_6$ und $R^*_6$ unabhängig voneinander Wasserstoff, $C_1$-$C_{18}$-Alkyl, $C_3$-$C_4$-Alkenyl, Benzyl oder ein Rest der Formel -$CH(R_9)$-$CH(R_{10})$-S-$R_{11}$ sind, sowie
$R_7$ und $R_8$ Wasserstoff bedeuten oder zusammen eine zweiwertige Gruppe

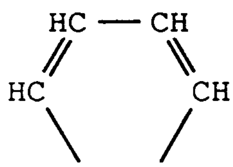

bilden,
$R_9$ und $R_{10}$ unabhängig voneinander Wasserstoff, Phenyl oder $C_1$-$C_{12}$-Alkyl sind,
$R_{11}$ $C_4$-$C_{18}$-Alkyl, Phenyl oder -$(CH_2)_a$-CO-$OR_{12}$ bedeutet,
a 1 oder 2 ist, und
$R_{12}$ für $C_1$-$C_{18}$-Alkyl steht.

$C_1$-$C_{12}$-Alkyl bzw. $C_1$-$C_{18}$-Alkyl, $C_4$-$C_{18}$-Alkyl oder $C_1$-$C_{25}$-Alkyl bedeutende Alkylreste ($R_1$ bis $R^*_6$ und $R_9$ bis $R_{12}$) können gerad- oder verzweigtkettig sein und sind je nach der bezeichneten Zahl der C-Atome beispielsweise

Methyl, Ethyl, Propyl, Isopropyl, n-Butyl, Isobutyl, t-Butyl, Pentyl, Isopentyl, Hexyl, Heptyl, Octyl, 2-Ethylhexyl, Nonyl, Decyl, Undecyl, Dodecyl, Tridecyl, Tetradecyl, Pentadecyl, Hexadecyl, Heptadecyl, Octadecyl, 2-Ethylbutyl, 1-Methylpentyl, 1,3-Dimethylbutyl, 1,1,3,3-Tetramethylbutyl, 1-Methylhexyl, Isoheptyl, 1-Methylheptyl, 1,1,3-Trimethylhexyl, 1-Methylundecyl, Eicosyl oder Henicosyl.

Bedeuten diese Reste weiter unten Gruppen mit einer geringeren Zahl von C-Atomen, so sind entsprechende Beispiele ebenfalls obiger Liste zu entnehmen.

Die Alkylreste außer $R_2$, $R_4$, $R^*_4$, $R_5$, $R^*_5$ und $R_{11}$ haben bevorzugt 1-12, insbesondere 1-6, vor allem 1-4 C-Atome. $R_2$, $R_4$, $R^*_4$, $R_5$ und $R^*_5$ haben als Alkyl vorzugsweise mehr als 3 C-Atome, $R_3$, $R_9$ und $R_{10}$ 1 bis 6 C-Atome.

$C_5$-$C_{12}$-Cycloalkyl bedeutet beispielsweise Cyclopentyl, Cyclohexyl, Cyclooctyl, Cyclododecyl, (bevorzugt sind Cycloalkylreste mit 5-8, insbesondere 5 oder 6 C-Atomen, besonders Cyclohexyl).

$C_3$-$C_4$-Alkenyl kann z.B. Allyl oder Methallyl sein.

Vorteilhaft sind durch die oben angegebene Reaktion erhältliche Produkte, wenn

$R_1$ Wasserstoff oder $C_1$-$C_{12}$-Alkyl,
$R_2$ $C_1$-$C_{18}$-Alkyl oder Phenyl,
$R_3$ Wasserstoff oder $C_1$-$C_6$-Alkyl,
$R_4$, $R^*_4$, $R_5$, $R^*_5$ Wasserstoff, $C_4$-$C_{18}$-Alkyl, Cyclohexyl, Benzyl, $\alpha$-Methylbenzyl oder $\alpha,\alpha$-Dimethylbenzyl und
$R_6$ und $R^*_6$ unabhängig voneinander Wasserstoff, Methyl, Allyl oder Benzyl sind.

Bevorzugte Produkte erhält man, wenn

$R_1$ Wasserstoff oder $C_1$-$C_4$-Alkyl,
$R_2$ $C_1$-$C_{18}$-Alkyl und
$R_3$ Wasserstoff oder $C_1$-$C_4$-Alkyl sind.

Besonders bevorzugte Produkte erhält man, wenn

$R_1$ und $R_3$ Wasserstoff oder Methyl und
$R_2$ $C_1$-$C_{12}$-Alkyl sind.

Man erhält auch besonders bevorzugte Produkte, wenn

$R_4$, $R^*_4$, $R_5$, und $R^*_5$ Wasserstoff sind.

Als Olefine der Formel I eignen sich vor allem alpha-Olefine mit 1 bis 20 C-Atomen, besonders Diisobutylen, d.h. $R_3$ ist vorzugsweise Wasserstoff.

Die weiteren Merkmale der Zusammensetzungen sind weitgehend durch die Zusammensetzung der Reaktionsmischung sowie die gewählten Verfahrensbedingungen bestimmt.

Die Ausgangsmischungen können z. B. wie folgt zusammengesetzt sein:
Das Molverhältnis Verbindung der Formel II: Verbindung der Formel III liegt zweckmäßig bei 5 : 95 bis 95 : 5, vorteilhaft bei 70 : 30 bis 30 : 70, besonders bei 70 : 30 bis 95 : 5 und ganz besonders bei 80 : 20 bis 90 : 10.

Die Verbindung der Formel I wird zweckmäßig in einer Menge von 0.5 bis 4 Mol pro Mol der Mischung aus den Verbindungen der Formeln II und III eingesetzt,
vorteilhaft in einer Menge von 1 bis 3, besonders 1 bis 2 Mol pro Mol der Mischung aus den Verbindungen der Formeln II und III.

Der saure Katalysator kann beispielsweise in einer Menge von 1-50, besonders 5 - 20, ganz besonders 7-15 Gewichts%, bezogen auf die Mischung aus den Verbindungen der Formeln II und III eingesetzt werden, oder, wenn es sich um eine Brønsted oder Lewis Säure handelt, zu 0.002 bis 10 mol%, vorzugsweise zu 0.1 bis 5 mol%.

Er ist bevorzugt eine saure Erde vom Typ der Bentonite oder Montmorillonite, wie weiter unten näher beschrieben.

Die Reaktion kann mit oder ohne Lösungs- oder Verdünnungsmittel durchgeführt werden, bevorzugt ohne. Es kann jedoch zweckmäßig sein, z.B. zur Abführung von Reaktionswärme ein Lösungs- oder Verdünnungsmittel einzusetzen. Wenn ein Lösungsmittel verwandt wird, sollte es bei Reaktionsbedingungen inert sein und einen geeigneten Siedepunkt haben. Geeignete Lösungsmittel sind beispielsweise gegegebenenfalls halogenierte Kohlenwasserstoffe, polare aprotische Lösungsmittel und Alkohole. Beispielhaft seien Petroletherfraktionen, bevorzugt höhersiedende, Toluol, Dichlorbenzol; Tetrahydrofuran (THF), Dimethylformamid (DMF), Dimethylacetamid (DMA), Hexamethylphosphorsäuretrisamid (HMPTA), Dimethylsulfoxid (DMSO) und Tetramethylharnstoff (TMU), ferner Pyridin oder Alkylpyridine; sowie Alkohole wie Butanol, Glykol oder Diethylenglykol genannt.

Die Reaktionstemperatur kann in einem weiten Bereich liegen, z.B. bei 60 bis 250°C, vorzugsweise bei 110 bis 200°C, besonders bevorzugt bei 130 bis 195°C und ganz besonders bevorzugt bei 160 bis 180°C.

Die Reaktion kann homogen oder heterogen katalysiert werden.

Als Katalysatoren eignen sich Brønsted-Säuren, Lewis-Säuren, Aluminiumsilikate, Ionen-austauscherharze, Zeolithe, natürlich vorkommende Schichtsilikate (z.B. "saure Erden" bzw. "acid clays", wie etwa Fuller's Earth) oder modifizierte Schichtsilikate.

Geeignete Brønsted-Säuren sind beispielsweise Säuren von anorganischen oder organischen Salzen, wie z.B. Salzsäure, Schwefelsäure, Phosphorsäure, Methansulfonsäure, p-Toluolsulfonsäure oder Carbonsäuren wie z.B. Essigsäure. Besonders geeignet ist die p-Toluolsulfonsäure.

Geeignete Lewis-Säuren sind beispielsweise Zinntetrachlorid, Aluminiumchlorid, Zinkchlorid oder Bortrifluorid-etherat. Zinntetrachlorid und Aluminiumchlorid sind speziell geeignet.

Geeignete Aluminiumsilikate sind beispielsweise solche, die in der Petrochemie weit verbreitet sind und auch als amorphe Aluminiumsilikate bezeichnet werden. Diese Verbindungen enthalten ca. 10-30 % Siliciumoxid und 70-90 % Aluminiumoxid. Ein besonders geeignetes Aluminiumsilikat ist HA-HPV® von Ketjen (Akzo).

Geeignete Ionenaustauscherharze sind beispielsweise Styrol-Divinylbenzol-Harze, die Sulfosäuregruppen tragen, wie z.B. Amberlite 200® und Amberlyst® von Rohm und Haas oder Dowex 50® von Dow Chemicals; perfluorierte Ionenaustauscherharze, wie z.B. Nafion H® von DuPont; oder andere supersaure Ionenaustauscherharze wie von T. Yamaguchi, Applied Catalysis, 61, 1-25 (1990) oder M. Hino et al., J. Chem. Soc. Chem. Commun. 1980, 851-852 beschrieben.

Geeignete Zeolithe sind beispielsweise solche, die in der Petrochemie als Cracking-Katalysatoren weit verbreitet sind und als kristalline Silicium-Aluminiumoxide mit unterschiedlicher Kristallstruktur bekannt sind. Besonders geeignet sind die Faujasite der Firma Union Carbide wie beispielsweise Zeolith X®, Zeolith Y® und ultrastabiler Zeolith Y®; Zeolith Beta® und Zeolith ZSM-12® von der Firma Mobil Oil Co.; und Zeolith Mordenit® von der Firma Norton.

Geeignete natürlich vorkommende Schichtsilikate werden auch als "Saure Erden" bezeichnet und sind beispielsweise Bentonite oder Montmorillonite, die großtechnisch abgebaut, gemahlen, mit Mineralsäuren behandelt und kalziniert werden. Besonders geeignete natürlich vorkommende Schichtsilikate sind die Fulcat®-Typen der Firma Laporte Adsorbents Co., wie z.B. Fulcat 22A®, Fulcat 22B®, Fulcat 20® oder Fulcat 40®; oder die Fulmont®-Typen der Firma Laporte Adsorbents Co., wie z.B. Fulmont XMP-3® oder Fulmont XMP-4®. Ein speziell bevorzugter Katalysator für das erfindungsgemäße Verfahren ist Fulcat 22B®, ein säureaktivierter Montmorillonit mit 4% freier Feuchtigkeit und einem Säuretiter von 20mg KOH/g. Die anderen Fulcat®-Typen und Fulmont®-Typen sind aber ebenfalls in diese bevorzugte Klasse einzuordnen, weil es nur geringfügige Unterschiede zwischen den einzelnen Typen gibt, wie beispielsweise in der Anzahl der sauren Zentren.

Modifizierte Schichtsilikate werden auch als "Pillared Clays" bezeichnet und leiten sich von den oben beschriebenen natürlich vorkommenden Schichtsilikaten ab, indem sie zwischen den Silicatschichten noch Oxide von beispielsweise Zirkon, Eisen, Zink, Nickel, Chrom, Cobalt oder Magnesium oder Elementen der seltenen Erden enthalten. Dieser Katalysator-Typ ist in der Literatur, wie z.B. von J. Clark et al., J. Chem. Soc. Chem. Commun. 1989, 1353-1354 beschrieben, weit verbreitet, wird aber nur von sehr wenigen Firmen hergestellt. Besonders bevorzugte modifizierte Schichtsilikate sind beispielsweise Envirocat EPZ-10®, Envirocat EPZG® oder Envirocat EPIC® von der Firma Contract Chemicals.

Die erfindungsgemäßen Reaktionsprodukte eignen sich hervorragend zum Stabilisieren von organischen Materialien gegen lichtinduzierten, thermischen oder /und oxidativen Abbau. Gegenstand der Erfindung sind daher auch Zusammensetzungen enthaltend ein gegen solche Abbaureaktionen empfindliches organisches Material und das erfindungsgemäße Reaktionsprodukt bzw. die Verwendung von erfindungsgemäßen Reaktionsprodukten als Stabilisatoren für organische Materialien gegen die genannten Abbauarten.

Die erfindungsgemäßen Reaktionsprodukte können insbesondere als Stabilisatoren für natürliche, halbsynthetische oder synthetische Polymere, besonders thermoplastische Kunststoffe und Elastomere, sowie für funktionelle Flüssigkeiten, insbesondere Schmierstoffe, Metallbearbeitungs- und Hydraulikflüssigkeiten, eingesetzt werden. Beispiele für solche Substrate sind der folgenden Aufzählung von geeigneten Materialien zu entnehmen.

1. Polymere von Mono- und Diolefinen, beispielsweise Polypropylen, Polyisobutylen, Polybuten-1, Poly-4-methylpenten-1, Polyisopren oder Polybutadien sowie Polymerisate von Cycloolefinen wie z.B. von Cyclopenten oder Norbornen; ferner Polyethylen (das gegebenenfalls vernetzt sein kann), z.B. Polyethylen hoher Dichte (HDPE), Polyethylen niederer Dichte (LDPE), lineares Polyethylen niederer Dichte (LLDPE), verzweigtes Polyethylen niederer Dichte (VLDPE).

Polyolefine, d.h. Polymere von Monoolefinen, wie sie beispielhaft im vorstehenden Absatz erwähnt sind, insbesondere Polyethylen und Polypropylen, können nach verschiedenen Verfahren hergestellt werden, insbesondere nach den folgenden Methoden:

a) radikalisch (gewöhnlich bei hohem Druck und hoher Temperatur).

b) mittels Katalysator, wobei der Katalysator gewöhnlich ein oder mehrere Metalle der Gruppe IVb, Vb, VIb oder VIII enthält. Diese Metalle besitzen gewöhnlich einen oder mehrere Liganden wie Oxide, Halogenide, Alkoholate, Ester, Ether, Amine, Alkyle, Alkenyle und/oder Aryle, die entweder $\pi$- oder $\sigma$-koordiniert sein können. Diese Metallkomplexe können frei oder auf Träger fixiert sein, wie beispielsweise auf aktiviertem Magnesiumchlorid, Titan(III)chlorid, Aluminiumoxid oder Siliziumoxid. Diese Katalysatoren können im Polymerisationsmedium löslich oder unlöslich sein. Die Katalysatoren können als solche in der Polymerisation aktiv sein, oder es können weitere Aktivatoren verwendet werden, wie beispielsweise Metallalkyle, Metallhydride, Metallalkylhalogenide, Metallalkyloxide oder Metallalkyloxane, wobei die Metalle Elemente der Gruppen Ia, IIa und/oder IIIa sind. Die Aktivatoren können beipielsweise mit weiteren Ester-, Ether-, Amin- oder Silylether-Gruppen modifiziert sein. Diese Katalysatorsysteme werden gewöhnlich als Phillips, Standard Oil Indiana, Ziegler (-Natta), TNZ (DuPont), Metallocen oder Single Site Katalysatoren (SSC) bezeichnet.

2. Mischungen der unter 1) genannten Polymeren, z.B. Mischungen von Polypropylen mit Polyisobutylen, Polypropylen mit Polyethylen (z.B. PP/HDPE, PP/LDPE) und Mischungen verschiedener Polyethylentypen (z.B. LDPE/HDPE).

3. Copolymere von Mono- und Diolefinen untereinander oder mit anderen Vinylmonomeren, wie z.B. Ethylen-Propylen-Copolymere, lineares Polyethylen niederer Dichte (LLDPE) und Mischungen desselben mit Polyethylen niederer Dichte (LDPE), Propylen-Buten-1-Copolymere, Propylen-Isobutylen-Copolymere, Ethylen-Buten-1-Copolymere, Ethylen-Hexen-Copolymere, Ethylen-Methylpenten-Copolymere, Ethylen-Hepten-Copolymere, Ethylen-Octen-Copolymere, Propylen-Butadien-Copolymere, Isobutylen-Isopren-Copolymere, Ethylen-Alkylacrylat-Copolymere, Ethylen-Alkylmethacrylat- Copolymere, Ethylen-Vinylacetat-Copolymere und deren Copolymere mit Kohlenmonoxid, oder Ethylen-Acrylsäure-Copolymere und deren Salze (Ionomere), sowie Terpolymere von Ethylen mit Propylen und einem Dien, wie Hexadien, Dicyclopentadien oder Ethylidennorbornen; ferner Mischungen solcher Copolymere untereinander und mit unter 1) genannten Polymeren, z.B. Polypropylen/Ethylen-Propylen-Copolymere, LDPE/Ethylen-Vinylacetat-Copolymere, LDPE/Ethylen-Acrylsäure-Copolymere, LLDPE/Ethylen-Vinylacetat-Copolymere, LLDPE/Ethylen-Acrylsäure-Copolymere und alternierend oder statistisch aufgebaute Polyalkylen/Kohlenmonoxid-Copolymere und deren Mischungen mit anderen Polymeren wie z.B. Polyamiden.

4. Kohlenwasserstoffharze (z.B. $C_5$-$C_9$) inklusive hydrierte Modifikationen davon (z.B. Klebrigmacherharze) und Mischungen von Polyalkylenen und Stärke.

5. Polystyrol, Poly-(p-methylstyrol), Poly-($\alpha$-methylstyrol).

6. Copolymere von Styrol oder $\alpha$-Methylstyrol mit Dienen oder Acrylderivaten, wie z.B. Styrol-Butadien, Styrol-Acrylnitril, Styrol-Alkylmethacrylat, Styrol-Butadien-Alkylacrylat und -methacrylat, Styrol-Maleinsäureanhydrid, Styrol-Acryl-nitril-Methylacrylat; Mischungen von hoher Schlagzähigkeit aus Styrol-Copolymeren und einem anderen Polymer, wie z.B. einem Polyacrylat, einem Dien-Polymeren oder einem Ethylen-Propylen-Dien-Terpolymeren; sowie Block-Copolymere des Styrols, wie z.B. Styrol-Butadien-Styrol, Styrol-Isopren-Styrol, Styrol-Ethylen/Butylen-Styrol oder Styrol-Ethylen/Propylen-Styrol.

7. Pfropfcopolymere von Styrol oder $\alpha$-Methylstyrol, wie z.B. Styrol auf Polybutadien, Styrol auf Polybutadien-Styrol- oder Polybutadien-Acrylnitril-Copolymere, Styrol und Acrylnitril (bzw. Methacrylnitril) auf Polybutadien; Styrol, Acrylnitril und Methylmethacrylat auf Polybutadien; Styrol und Maleinsäureanhydrid auf Polybutadien; Styrol, Acrylnitril und Maleinsäureanhydrid oder Maleinsäureimid auf Polybutadien; Styrol und Maleinsäureimid auf Polybutadien, Styrol und Alkylacrylate bzw. Alkylmethacrylate auf Polybutadien, Styrol und Acrylnitril auf Ethylen-Propylen-Dien-Terpolymeren, Styrol und Acrylnitril auf Polyalkylacrylaten oder Polyalkylmethacrylaten, Styrol und Acrylnitril auf Acrylat-Butadien-Copolymeren, sowie deren Mischungen mit den unter 6) genannten Copolymeren, wie sie z.B. als sogenannte ABS-, MBS-, ASA- oder AES-Polymere bekannt sind.

8. Halogenhaltige Polymere, wie z.B. Polychloropren, Chlorkautschuk, chloriertes oder chlorsulfoniertes Polyethylen, Copolymere von Ethylen und chloriertem Ethylen, Epichlorhydrinhomo- und -copolymere, insbesondere Polymere aus halogenhaltigen Vinylverbindungen, wie z.B. Polyvinylchlorid, Polyvinylidenchlorid, Polyvinylfluorid, Polyvinylidenfluorid; sowie deren Copolymere, wie Vinylchlorid-Vinylidenchlorid, Vinylchlorid-Vinylacetat oder Vinylidenchlorid-Vinylacetat.

9. Polymere, die sich von $\alpha$,$\beta$-ungesättigten Säuren und deren Derivaten ableiten, wie Polyacrylate und Polymethacrylate, mit Butylacrylat schlagzäh modifizierte Polymethylmethacrylate, Polyacrylamide und Polyacrylnitrile.

10. Copolymere der unter 9) genannten Monomeren untereinander oder mit anderen ungesättigten Monomeren, wie z.B. Acrylnitril-Butadien-Copolymere, Acrylnitril-Alkylacrylat-Copolymere, Acrylnitril-Alkoxyalkylacrylat-Copolymere, Acrylnitril-Vinylhalogenid-Copolymere oder Acrylnitril-Alkylmethacrylat-Butadien-Terpolymere.

11. Polymere, die sich von ungesättigten Alkoholen und Aminen bzw. deren Acylderivaten oder Acetalen ableiten, wie Polyvinylalkohol, Polyvinylacetat, -stearat, - benzoat, maleat, Polyvinylbutyral, Polyallylphthalat, Polyallylmelamin; sowie deren Copolymere mit in Punkt 1 genannten Olefinen.

12. Homo- und Copolymere von cyclischen Ethern, wie Polyalkylenglykole, Polyethylenoxyd, Polypropylenoxyd oder deren Copolymere mit Bisglycidylethern.

13. Polyacetale, wie Polyoxymethylen, sowie solche Polyoxymethylene, die Comonomere, wie z.B. Ethylenoxid, enthalten; Polyacetale, die mit thermoplastischen Polyurethanen, Acrylaten oder MBS modifiziert sind.

14. Polyphenylenoxide und -sulfide und deren Mischungen mit Styrolpolymeren oder Polyamiden.

15. Polyurethane, die sich von Polyethern, Polyestern und Polybutadienen mit endständigen Hydroxylgruppen einerseits und aliphatischen oder aromatischen Polyisocyanaten andererseits ableiten, sowie deren Vorprodukte, z.B. Polyetherpolyole.

16. Polyamide und Copolyamide, die sich von Diaminen und Dicarbonsäuren und/oder von Aminocarbonsäuren oder den entsprechenden Lactamen ableiten, wie Polyamid 4, Polyamid 6, Polyamid 6/6, 6/10, 6/9, 6/12, 4/6, 12/12, Polyamid 11, Polyamid 12, aromatische Polyamide ausgehend von m-Xylol, Diamin und Adipinsäure; Polyamide, hergestellt aus Hexamethylendiamin und Iso- und/oder Terephthalsäure und gegebenenfalls einem Elastomer als Modifikator, z.B. Poly-2,4,4-trimethylhexamethylenterephthalamid oder Poly-m-phenylen-isophthalamid. Block-Copolymere der vorstehend genannten Polyamide mit Polyolefinen, Olefin-Copolymeren, Ionomeren oder chemisch gebundenen oder gepfropften Elastomeren; oder mit Polyethern, wie z.B. mit Polyethylenglykol, Polypropylenglykol oder Polytetramethylenglykol. Ferner mit EPDM oder ABS modifizierte Polyamide oder Copolyamide; sowie während der Verarbeitung kondensierte Polyamide ("RIM-Polyamidsysteme").

17. Polyharnstoffe, Polyimide, Polyamid-imide und Polybenzimidazole.

18. Polyester, die sich von Dicarbonsäuren und Dialkoholen und/oder von Hydroxycarbonsäuren oder den entsprechenden Lactonen ableiten, wie Polyethylenterephthalat, Polybutylenterephthalat, Poly-1,4-dimethylolcyclohexanterephthalat, Polyhydroxybenzoate, sowie Block-Polyether-ester, die sich von Polyethern mit Hydroxylendgruppen ableiten; ferner mit Polycarbonaten oder MBS modifizierte Polyester.

19. Polycarbonate und Polyestercarbonate.

20. Polysulfone, Polyethersulfone und Polyetherketone.

21. Vernetzte Polymere, die sich von Aldehyden einerseits und Phenolen, Harnstoff oder Melamin andererseits ableiten, wie Phenol-Formaldehyd-, Harnstoff-Formaldehyd-und Melamin-Formaldehydharze.

22. Trocknende und nicht-trocknende Alkydharze.

23. Ungesättigte Polyesterharze, die sich von Copolyestern gesättigter und ungesättigter Dicarbonsäuren mit mehrwertigen Alkoholen, sowie Vinylverbindungen als Vernetzungsmittel ableiten, wie auch deren halogenhaltige, schwerbrennbare Modifikationen.

24. Vernetzbare Acrylharze, die sich von substituierten Acrylsäureestern ableiten, wie z.B. von Epoxyacrylaten, Urethan-acrylaten oder Polyester-acrylaten.

25. Alkydharze, Polyesterharze und Acrylatharze, die mit Melaminharzen, Harnstoffharzen, Polyisocyanaten oder Epoxidharzen vernetzt sind.

26. Vernetzte Epoxidharze, die sich von Polyepoxiden ableiten, z.B. von Bis-glycidylethern oder von cycloaliphatischen Diepoxiden.

27. Natürliche Polymere, wie Cellulose, Naturkautschuk, Gelatine, sowie deren polymerhomolog chemisch abgewandelte Derivate, wie Celluloseacetate, -propionate und -butyrate, bzw. die Celluloseether, wie Methylcellulose; sowie Kolophoniumharze und Derivate.

28. Mischungen (Polyblends) der vorgenannten Polymeren, wie z.B. PP/EPDM, Polyamid/EPDM oder ABS, PVC/EVA, PVC/ABS, PVC/MBS, PC/ABS, PBTP/ABS, PC/ASA, PC/PBT, PVC/CPE, PVC/Acrylate, POM/thermoplastisches PUR, PC/thermoplastisches PUR, POM/Acrylat, POM/MBS, PPO/HIPS, PPO/PA 6.6 und Copolymere, PA/HDPE, PA/PP, PA/PPO.

29. Natürliche und synthetische organische Stoffe, die reine monomere Verbindungen oder Mischungen von solchen darstellen, beispielsweise Mineralöle, tierische oder pflanzliche Fette, Öle und Wachse, oder Öle, Wachse und Fette auf Basis synthetischer Ester (z.B. Phthalate, Adipate, Phosphate oder Trimellitate), sowie Abmischungen synthetischer Ester mit Mineralölen in beliebigen Gewichtsverhältnissen, wie sie z.B. als Spinnpräparationen Anwendung finden, sowie deren wäßrige Emulsionen.

30. Wäßrige Emulsionen natürlicher oder synthetischer Kautschuke, wie z.B. Naturkautschuk-Latex oder Latices von carboxylierten Styrol-Butadien-Copolymeren.

In den erfindungsgemäßen Zusammensetzungen sind die erfindungsgemäßen Reaktionsprodukte zweckmäßig zu 0.01 bis 10, beispielsweise zu 0.05 bis 5, vorzugsweise zu 0.05 bis 3, insbesondere jedoch zu 0.1 bis 2 Gew.-% enthalten. Es kann sich dabei um eine oder mehrere dieser erfindungsgemäßen Reaktionsprodukte handeln, und die Gewichtsprozentangaben beziehen sich auf die gesamte Menge dieser erfindungsgemäßen Reaktionsprodukte. Berechnungsgrundlage ist dabei das Gesamtgewicht des organischen Materials ohne die erfindungsgemäßen Reaktionsprodukte.

Die Einarbeitung in die Materialien kann beispielsweise durch Einmischen oder Aufbringen der erfindungsgemäßen Reaktionsprodukte und gegebenenfalls weiterer Additive nach den in der Technik üblichen Methoden erfolgen. Handelt es sich um Polymere, insbesondere synthetische Polymere, kann die Einarbeitung vor oder während der Formgebung, oder durch Aufbringen der gelösten oder dispergierten erfindungsgemäßen Reaktionsprodukte auf das Polymere, gegebenenfalls unter nachträglichem Verdunsten des Lösungsmittels erfolgen. Im Fall von Elastomeren können diese auch als Latices stabilisiert werden. Eine weitere Möglichkeit der Einarbeitung der erfindungsgemäßen Reaktionsprodukte in Polymere besteht in deren Zugabe vor, während oder unmittelbar nach der Polymerisation der entsprechenden Monomeren bzw. vor der Vernetzung. Die erfindungsgemäßen Reaktionsprodukte können dabei als solche, aber auch in encapsulierter Form (z.B in Wachsen, Ölen oder Polymeren) zugesetzt werden. Im Falle der Zugabe vor oder während der Polymerisation können die erfindungsgemäßen Reaktionsprodukte auch als Regulatoren für die Kettenlänge der Polymeren (Kettenabbrecher) wirken.

Die erfindungsgemäßen Reaktionsprodukte oder Mischungen davon können auch in Form eines Masterbatches, der diese Verbindungen beispielsweise in einer Konzentration von 2.5 bis 25 Gew.-% enthält, den zu stabilisierenden Kunststoffen zugesetzt werden.

Zweckmäßig kann die Einarbeitung der erfindungsgemäßen Reaktionsprodukte nach folgenden Methoden erfolgen:

- als Emulsion oder Dispersion (z.B. zu Latices oder Emulsionspolymeren)
- Als Trockenmischung während des Vermischens von Zusatzkomponenten oder Polymermischungen
- durch direktes Zugeben in die Verarbeitungsapparatur (z.B. Extruder, Innenmischer usw.)
- als Lösung oder Schmelze.

Erfindungsgemäße Polymerzusammensetzungen können in verschiedener Form angewandt bzw. zu verschiedenen Produkten verarbeitet werden, z.B. als (zu) Folien, Fasern, Bändchen, Formmassen, Profilen, oder als Bindemittel für Lacke, Klebstoffe oder Kitte.

Die Erfindung betrifft auch ein Verfahren zum Stabilisieren von organischem Material, insbesondere von thermoplastischen Polymeren, Elastomeren oder funktionellen Flüssigkeiten, vor allem Schmierstoffen, gegen oxidativen, thermischen und/oder lichtinduzierten Abbau, dadurch gekennzeichnet, daß man diesem Material als Stabilisatoren erfindungsgemäßen Reaktionsprodukte zusetzt bzw. auf dieses aufbringt.

Die erfindungsgemäßen Reaktionsprodukte sind beispielsweise besonders geeignet, um funktionellen Flüssigkeiten verbesserte Gebrauchseigenschaften zu verleihen. Es ist dabei insbesondere auf ihre überraschend gute antioxidative und schlammverhindernde (deposit control) Wirkung hinzuweisen. Deshalb umfaßt die Erfindung auch Zusammensetzungen, enthaltend eine funktionelle Flüssigkeit und wenigstens eine Verbindung der allgemeinen Formel I, wie oben beschrieben.

Als funktionelle Flüssigkeiten sind beispielsweise Schmierstoffe, Hydraulikflüssigkeiten und Metallbearbeitungsflüssigkeiten zu nennen. Schmierfette sollen hier ebenfalls unter funktionelle Flüssigkeiten fallen.

Die in Frage kommenden Schmierstoffe basieren beispielsweise auf mineralischen oder synthetischen Ölen oder Mischungen davon oder auf pflanzlichen und tierischen Ölen, Fetten und Wachsen. Die Schmierstoffe sind dem Fachmann geläufig und in der einschlägigen Fachliteratur, wie beispielsweise in Dieter Klamann, "Schmierstoffe und verwandte Produkte" (Verlag Chemie, Weinheim, 1982), in Schewe-Kobek, "Das Schmiermittel-Taschenbuch" (Dr. Alfred Hüthig-Verlag, Heidelberg, 1974) und in "Ullmanns Enzyklopädie der technischen Chemie", Bd. 13, Seiten 85-94 (Verlag Chemie, Weinheim, 1977) beschrieben.

Die Schmierstoffe sind insbesondere Öle und Fette, beispielsweise basierend auf einem Mineralöl. Bevorzugt sind Öle.

Die Mineralöle basieren insbesondere auf Kohlenwasserstoffverbindungen.

Beispiele von synthetischen Schmierstoffen umfassen Schmierstoffe auf der Basis der aliphatischen oder aromatischen Carboxylester, der polymeren Ester, der Polyalkylenoxide, der Phosphorsäureester, der Poly-$\alpha$-olefine oder der Silicone, eines Diesters einer zweiwertigen Säure mit einem einwertigen Alkohol, wie z.B. Dioctylsebacat oder Dinonyladipat, eines Triesters von Trimethylolpropan mit einer einwertigen Säure oder mit einem Gemisch solcher Säuren, wie z.B. Trimethylolpropantripelargonat, Trimethylolpropan-tricaprylat oder Gemische davon, eines Tetraesters von Pentaerythrit mit einer einwertigen Säure oder mit einem Gemisch solcher Säuren, wie z.B. Pentaerythrit-tetracaprylat, oder eines komplexen Esters von einwertigen und zweiwertigen Säuren mit mehrwertigen Alkoholen, z.B. ein komplexer Ester von Trimethylolpropan mit Capryl- und Sebacinsäure oder von einem Gemisch davon. Besonders geeignet sind neben Mineralölen z.B. Poly-$\alpha$-Olefine, Schmierstoffe auf Esterbasis, Phosphate, Glykole, Polyglykole und Polyalkylenglykole, sowie deren Mischungen mit Wasser.

Als pflanzliche Schmierstoffe sind die Öle, Fette und Wachse, gewonnen beispielsweise aus Oliven, Palmen, Palmkernen, Rüben, Raps, Leinsamen, Nüssen, Soja, Baumwolle, Ricinus, Sonnenblumen, Kürbiskernen, Kokos, Mais oder deren modifizierte Formen, z.B. geschwefelte oder epoxidierte Öle, wie epoxidiertes Sojaöl, sowie Mischungen der Substanzen zu nennen. Beispiele für tierische Öle, Fette und Wachse, die als Schmierstoffe angewandt werden können, sind Talge, Fischöle, Spermöl, Klauenöl, Trane und Specköle, deren modifizierte Formen und Mischungen.

Metallbearbeitungsflüssigkeiten wie Walz-, Zieh- und Schneidöle basieren zumeist auf den oben beschriebenen Mineral- und synthetischen Ölen und können auch als Öl-in-Wasser- bzw. Wasser-in-Öl-Emulsionen vorliegen. Das gleiche gilt für Hydraulikflüssigkeiten. Als weitere funktionelle Flüssigkeiten kommen z.B. Kompressoröle und Spinnpräparationen in Frage.

Die erfindungsgemäßen Reaktionsprodukte, wie oben beschrieben, können z.B. in Mengen von 0.01 bis 10 Gew.-%, zweckmäßig in Mengen von 0.05 bis 5 Gew.-%, vorzugsweise in einer Menge von 0.05 bis 3 Gew.-% und ganz besonders bevorzugt von

0.5 bis 1.5 Gew.-%, bezogen auf die Zusammensetzung, in der funktionellen Flüssigkeit vorliegen.

Die erfindungsgemäßen Reaktionsprodukte können der funktionellen Flüssigkeit auf an sich bekannte Weise beigemischt werden. Die Verbindungen sind beispielsweise in Ölen gut löslich. Es ist auch möglich, einen sogenannten Masterbatch herzustellen, der nach Maßgabe des Verbrauchs auf Einsatzkonzentrationen mit der entsprechenden funktionellen Flüssigkeit verdünnt werden kann.

Zusätzlich zu den erfindungsgemäßen Produkten bzw. Mischungen können die erfindungsgemäßen Zusammensetzungen, insbesondere wenn sie organische, vorzugsweise synthetische, Polymere enthalten, noch weitere übliche Additive enthalten. Beispiele für solche Additive sind:

1. Antioxidantien

1.1. Alkylierte Monophenole, z.B. 2,6-Di-tert-butyl-4-methylphenol, 2-butyl-4,6-dimethylphenol, 2,6-Di-tert-butyl-4-ethylphenol, 2,6-Di-tert-butyl-4-n-butylphenol, 2,6-Di-tert-butyl-4-iso-butylphenol, 2,6-Di-cyclopentyl-4-methylphenol, 2-(α-Methylcyclohexyl)-4,6-dimethylphenol, 2,6-Di-octadecyl-4-methylphenol, 2,4,6-Tri-cyclohexylphenol, 2,6-Di-tert-butyl-4-methoxymethylphenol, 2,6-Di-nonyl-4-methylphenol, 2,4-Dimethyl-6-(1'-methyl-undec-1'-yl)-phenol, 2,4-Dimethyl-6-(1'-methyl-heptadec-1'-yl)-phenol, 2,4-Dimethyl-6-(1'-methyl-tridec-1'-yl)-phenol und Mischungen davon.

1.2. Alkylthiomethylphenole, z.B. 2,4-Di-octylthiomethyl-6-tert-butylphenol, 2,4-Di-octylthiomethyl-6-methylphenol, 2,4-Di-octylthiomethyl-6-ethylphenol, 2,6-Di-dodecylthiomethyl-4-nonylphenol.

1.3. Hydrochinone und alkylierte Hydrochinone, z.B. 2,6-Di-tert-butyl-4-methoxyphenol, 2,5-Di-tert-butyl-hydrochinon, 2,5-Di-tert-amyl-hydrochinon, 2,6-Diphenyl-4-octadecyloxyphenol, 2,6-Di-tert-butyl-hydrochinon, 2,5-Di-tert-butyl-4-hydroxyanisol, 3,5-Di-tert-butyl-4-hydroxyanisol, 3,5-Di-tert-butyl-4-hydroxyphenyl-stearat, Bis-(3,5-di-tert-butyl-4-hydroxyphenyl)adipat.

1.4. Tocopherole, z.B. α-Tocopherol, β-Tocopherol, γ-Tocopherol, δ-Tocopherol und Mischungen davon (Vitamin E).

1.5. Hydroxylierte Thiodiphenylether, z.B. 2,2'-Thio-bis-(6-tert-butyl-4-methylphenol), 2,2'-Thio-bis-(4-octylphenol), 4,4'-Thio-bis-(6-tert-butyl-3-methylphenol), 4,4'-Thio-bis-(6-tert-butyl-2-methylphenol), 4,4'-Thio-bis-(3,6-di-sec.-amylphenol), 4,4'-Bis-(2,6-dimethyl-4-hydroxyphenyl)-disulfid.

1.6. Alkyliden-Bisphenole, z.B. 2,2'-Methylen-bis-(6-tert-butyl-4-methylphenol) 2,2'-Methylen-bis-(6-tert-butyl-4-ethylphenol), 2,2'-Methylen-bis-[4-methyl-6-(α-methylcyclohexyl)-phenol], 2,2'-Methylen-bis-(4-methyl-6-cyclohexylphenol), 2,2'-Methylen-bis-(6-nonyl-4-methylphenol), 2,2'-Methylen-bis-(4,6-di-tert-butylphenol), 2,2'-Ethyliden-bis-(4,6-di-tert-butylphenol), 2,2'-Ethyliden-bis-(6-tert-butyl-4-isobutylphenol), 2,2'-Methylen-bis-[6-(α-methylbenzyl)-4-nonylphenol], 2,2'-Methylen-bis-[6-(α,α-dimethylbenzyl)-4-nonylphenol], 4,4'-Methylen-bis-(2,6-di-tert-butylphenol), 4,4'-Methylen-bis-(6-tert-butyl-2-methylphenol), 1,1-Bis-(5-tert-butyl-4-hydroxy-2-methylphenyl)-butan, 2,6-Bis-(3-tert-butyl-5-methyl-2-hydroxybenzyl)-4-methylphenol, 1,1,3-Tris-(5-tert-butyl-4-hydroxy-2-methylphenyl)-butan, 1,1-Bis-(5-tert-butyl-4-hydroxy-2-methylphenyl)-3-n-dodecylmercaptobutan, Ethylenglycol-bis-[3,3-bis-(3'-tert-butyl-4'-hydroxyphenyl)-butyrat], Bis-(3-tert-butyl-4-hydroxy-5-methyl-phenyl)-dicyclopentadien, Bis-[2-(3'-tert-butyl-2'-hydroxy-5'-methyl-benzyl)-6-tert-butyl-4-methyl-phenyl]-terephthalat, 1,1-Bis-(3,5-dimethyl-2-hydroxyphenyl)-butan,2,2-Bis-(3,5-di-tert-butyl-4-hydroxyphenyl)-propan, 2,2-Bis-(5-tert-butyl-4-hydroxy-2-methylphenyl)-4-n-dodecylmercaptobutan, 1,1,5,5-Tetra-(5-tert-butyl-4-hydroxy-2-methylphenyl)-pentan.

1.7. O-, N- und S-Benzylverbindungen, z.B. 3,5,3',5'-Tetra-tert-butyl-4,4'-dihydroxydibenzylether, Octadecyl-4-hydroxy-3,5-dimethylbenzyl-mercaptoacetat, Tris-(3,5-di-tert-butyl-4-hydroxybenzyl)-amin, Bis-(4-tert-butyl-3-hydroxy-2,6-dimethylbenzyl)-di-thioterephthalat, Bis-(3,5-di-tert-butyl4-hydroxybenzyl)-sulfid, Isooctyl-3,5-di-tert-butyl-4-hydroxybenzyl-mercaptoacetat.

1.8. Hydroxybenzylierte Malonate, z.B. Dioctadecyl-2,2-bis-(3,5-di-tert-butyl-2-hydroxybenzyl)-malonat, Di-octadecyl-2-(3-tert-butyl-4-hydroxy-5-methylbenzyl)-malonat, Di-dodecylmercaptoethyl-2,2-bis-(3,5-di-tert-butyl-4-hydroxybenzyl)-malonat, Di-[4-(1,1,3,3-tetramethylbutyl)-phenyl]-2,2-bis-(3,5-di-tert-butyl-4-hydroxybenzyl)-malonat.

1.9. Hydroxybenzyl-Aromaten, z.B. 1,3,5-Tris-(3,5-di-tert-butyl-4-hydroxybenzyl)-2,4,6-trimethylbenzol, 1,4-Bis-(3,5-di-tert-butyl-4-hydroxybenzyl)-2,3,5,6-tetramethylbenzol, 2,4,6-Tris-(3,5-di-tert-butyl-4-hydroxybenzyl)-phenol.

1.10. Triazinverbindungen, z.B. 2,4-Bis-octylmercapto-6-(3,5-di-tert-butyl-4-hydroxyanilino)-1,3,5-triazin, 2-Octylmercapto-4,6-bis-(3,5-di-tert-butyl-4-hydroxyanilino)-1,3,5-triazin, 2-Octylmercapto-4,6-bis-(3,5-di-tert-butyl-4-hydroxyphenoxy)-1,3,5-triazin, 2,4,6-Tris-(3,5-di-tert-butyl-4-hydroxyphenoxy)-1,2,3-triazin, 1,3,5-Tris-(3,5-di-tert-butyl-4-hydroxybenzyl)-isocyanurat, 1,3,5-Tris-(4-tert-butyl-3-hydroxy-2,6-dimethylbenzyl)-isocyanurat, 2,4,6-Tris-(3,5-di-tert-butyl-4-hydroxyphenylethyl)-1,3,5-triazin, 1,3,5-Tris-(3,5-di-tert-butyl-4-hydroxyphenylpropionyl)-hexahydro-1,3,5-triazin, 1,3,5-Tris-(3,5-dicyclohexyl-4-hydroxybenzyl)-isocyanurat.

1.11. Benzylphosphonate, z.B. Dimethyl-2,5-di-tert-butyl-4-hydroxybenzylphosphonat, Diethyl-3,5-di-tert-butyl-4-hydroxybenzylphosphonat, Dioctadecyl-3,5-di-tert-butyl-4-hydroxybenzylphosphonat, Dioctadecyl-5-tert-butyl-4-hydroxy-3-methylbenzylphosphonat, Ca-Salz des 3,5-Di-tert-butyl-4-hydroxybenzyl-phosphonsäure-monoethylesters.

1.12. Acylaminophenole, z.B. 4-Hydroxy-laurinsäureanilid, 4-Hydroxystearinsäureanilid, N-(3,5-di-tert-butyl-4-hydroxyphenyl)-carbaminsäureoctylester.

1.13. Ester der β-(3,5-Di-tert-butyl-4-hydroxyphenyl)-propionsäure mit ein- oder mehrwertigen Alkoholen, wie z.B. mit Methanol, Ethanol, Octanol, Octadecanol, 1,6-Hexandiol, 1,9-Nonandiol, Ethylenglycol, 1,2-Propandiol, Neopentylglycol, Thiodiethylenglycol, Diethylenglycol, Triethylenglycol, Pentaerythrit, Tris-(hydroxyethyl)-isocyanurat, N,N'-Bis-(hydroxyethyl)-oxalsäurediamid, 3-Thiaundecanol, 3-Thiapentadecanol, Trimethylhexandiol, Trimethylolpropan, 4-Hydroxymethyl-1-phospha-2,6,7-trioxabicyclo-[2.2.2]-octan.

1.14. Ester der β-(5-tert-Butyl-4-hydroxy-3-methylphenyl)-propionsäure mit ein- oder mehrwertigen Alkoholen, wie z.B. mit Methanol, Ethanol, Octanol, Octadecanol, 1,6-Hexandiol, 1,9-Nonandiol, Ethylenglycol, 1,2-Propandiol, Neopentylglycol, Thiodiethylenglycol, Diethylenglycol, Triethylenglycol, Pentaerythrit, Tris-(hydroxy)ethyl-isocyanurat, N,N'-Bis-(hydroxyethyl)-oxalsäurediamid, 3-Thiaundecanol, 3-Thiapentadecanol, Trimethylhexandiol, Trimethylolpropan, 4-Hydroxymethyl-1-phospha-2,6,7-trioxabicyclo-[2.2.2]-octan.

1.15. Ester der β-(3,5-Dicyclohexyl-4-hydroxyphenyl)-propionsäure mit ein-oder mehrwertigen Alkoholen, wie z.B. mit Methanol, Ethanol, Octanol, Octadecanol, 1,6-Hexandiol, 1,9-Nonandiol, Ethylenglycol, 1,2-Propandiol, Neopentylglycol, Thiodiethylenglycol, Diethylenglycol, Triethylenglycol, Pentaerythrit, Tris-(hydroxy)ethyl-isocyanurat, N,N'-Bis-(hydroxyethyl)-oxalsäurediamid, 3-Thiaundecanol, 3-Thiapentadecanol, Trimethylhexandiol, Trimethylolpropan, 4-Hydroxymethyl-1-phospha-2,6,7-trioxabicyclo-[2.2.2]-octan.

1.16. Ester der 3,5-Di-tert-butyl-4-hydroxyphenylessigsäure mit ein- oder mehrwertigen Alkoholen, wie z.B. mit Methanol, Ethanol, Octanol, Octadecanol, 1,6-Hexandiol, 1,9-Nonandiol, Ethylenglycol, 1,2-Propandiol, Neopentylglycol, Thiodiethylenglycol, Diethylenglycol, Triethylenglycol, Pentaerythrit, Tris-(hydroxy)ethyl-isocyanurat, N,N'-Bis-(hydroxyethyl)-oxalsäurediamid, 3-Thiaundecanol, 3-Thiapentadecanol, Trimethylhexandiol, Trimethylolpropan, 4-Hydroxymethyl-1-phospha-2,6,7-trioxabicyclo-[2.2.2]-octan.

1.17. Amide der β-(3,5-Di-tert-butyl-4-hydroxyphenyl)-propionsäure, wie z.B. N,N'-Bis-(3,5-di-tert-butyl-4-hydroxyphenylpropionyl)-hexamethylendiamin, N,N'-Bis-(3,5-di-tert-butyl-4-hydroxyphenylpropionyl)-trimethylendiamin, N,N'-Bis-(3,5-di-tert-butyl-4-hydroxyphenylpropionyl)-hydrazin.

2. UV-Absorber und Lichtschutzmittel

2.1.2-(2'-Hydroxyphenyl)-benzotriazole, wie z.B. 2-(2'-Hydroxy-5'-methylphenyl)-benzotriazol, 2-(3',5'-Di-tert-butyl-2'-hydroxyphenyl)-benzotriazol, 2-(5'-tert-Butyl-2'-hydroxyphenyl)-benzotriazol, 2-(2'-Hydroxy-5'-(1,1,3,3-tetramethylbutyl)phenyl)-benzotriazol, 2-(3',5'-Di-tert-butyl-2'-hydroxyphenyl)-5-chlor-benzotriazol, 2-(3'-tert-Butyl-2'-hydroxy-5'-methylphenyl)-5-chlor-benzotriazol, 2-(3'-sec-Butyl-5'-tert-butyl-2'-hydroxyphenyl)-benzotriazol, 2-(2'-Hydroxy-4'-octoxyphenyl)-benzotriazol, 2-(3',5'-Di-tertamyl-2'-hydroxyphenyl)-benzotriazol, 2-(3',5'-Bis-(α,α-dimethylbenzyl)-2'-hydroxyphenyl)-benzotriazol, Mischung aus 2-(3'-tert-Butyl-2'-hydroxy-5'-(2-octyloxycarbonylethyl)-phenyl)-5-chlor-benzotriazol, 2-(3'-tert-Butyl-5'-[2-(2-ethylhexyloxy)-carbonylethyl]-2'-hydroxyphenyl)-5-chlor-benzotriazol, 2-(3'-tert-Butyl-2'-hydroxy-5'-(2-methoxycarbonyl-ethyl)phenyl)-5-chlor-benzotriazol, 2-(3'-tert-Butyl-2'-hydroxy-5'-(2-methoxycarbonyl-ethyl)phenyl)-benzotriazol, 2-(3'-tert-Butyl-2'-hydroxy-5'-(2-octyloxycarbonylethyl)phenyl)-benzotriazol, 2-(3'-tert-Butyl-5'-[2-(2-ethylhexyloxy)carbonylethyl]-2'-hydroxyphenyl)-benzotriazol, 2-(3'-Dodecyl-2'-hydroxy-5'-methylphenyl)-benzotriazol, und 2-(3'-tert-Butyl-2'-hydroxy-5'-(2-isooctyloxycarbonylethyl)phenyl-benzotriazol, 2,2'-Methylen-bis[4-(1,1,3,3-tetramethylbutyl)-6-benzotriazol-2-yl-phenol]; Umesterungsprodukt von 2-[3'-tert-Butyl-5'-(2-methoxycarbonylethyl)-2'-hydroxy-phenyl]-benzotriazol mit Polyethylenglycol 300; $[R\text{-}CH_2CH_2\text{-}COO(CH_2)3]_2$ mit R = 3'-tert-Butyl-4'-hydroxy-5'-2H-benzotriazol-2-yl-phenyl.

2.2. 2-Hydroxybenzophenone, wie z.B. das 4-Hydroxy-, 4-Methoxy-, 4-Octoxy-, 4-Decyloxy-, 4-Dodecyloxy-, 4-Benzyloxy-, 4,2',4'-Trihydroxy-, 2'-Hydroxy-4,4'-dimethoxy-Derivat.

2.3. Ester von gegebenenfalls substituierten Benzoesäuren, wie z.B. 4-tert-Butyl-phenylsalicylat, Phenylsalicylat, Octylphenyl-salicylat, Dibenzoylresorcin, Bis-(4-tert-butylbenzoyl)-resorcin, Benzoylresorcin, 3,5-Di-tert-butyl-4-hydroxybenzoesäure-2,4-di-tert-butylphenylester, 3,5-Di-tert-butyl-4-hydroxybenzoesäurehexadecylester, 3,5-Di-tert-butyl-4-hydroxybenzoesäure-octadecylester, 3,5-Di-tert-butyl-4-hydroxybenzoesäure-2-methyl-4,6-di-tert-butylphenylester.

2.4. Acrylate, wie z.B. α-Cyan-β,β-diphenylacrylsäure-ethylester bzw. - isooctylester, α-Carbomethoxy-zimtsäuremethylester, α-Cyano-β-methyl-p-methoxy-zimtsäuremethylester bzw. -butylester, α-Carbomethoxy-p-methoxy-zimtsäure-methylester, N-(β-Carbomethoxy-β-cyanovinyl)-2-methyl-indolin.

2.5. Nickelverbindungen, wie z.B. Nickelkomplexe des 2,2'-Thio-bis-[4-(1,1,3,3-tetramethylbutyl)-phenols], wie der 1:1- oder der 1:2-Komplex, gegebenenfalls mit zusätzlichen Liganden, wie n-Butylamin, Triethanolamin oder N-Cyclohexyl-diethanolamin, Nickeldibutyldithiocarbamat, Nickelsalze von 4-Hydroxy-3,5-di-tert-butylbenzylphosphonsäure-monoalkylestern, wie vom Methyl- oder Ethylester, Nickelkomplexe von Ketoximen, wie von 2-Hydroxy-4-methyl-phenyl-undecylketoxim, Nickelkomplexe des 1-Phenyl-4-lauroyl-5-hydroxy-pyrazols, gegebenenfalls mit zusätzlichen Liganden.

2.6. Sterisch gehinderte Amine, wie z.B. Bis-(2,2,6,6-tetramethyl-piperidyl)-sebacat, Bis-(2,2,6,6-tetramethyl-piperidyl)-succinat, Bis-(1,2,2,6,6-pentamethylpiperidyl)-sebacat, n-Butyl-3,5-di-tert-butyl-4-hydroxybenzyl-malonsäure-bis(1,2,2,6,6-pentamethylpiperidyl)-ester, Kondensationsprodukt aus 1-Hydroxyethyl-2,2,6,6-tetramethyl-4-hydroxypiperidin und Bernsteinsäure, Kondensationsprodukt aus N,N'-Bis-(2'2,6,6-Tetramethyl-4-piperidyl)-hexamethylendiamin und 4-tert-Octylamino-2,6-dichlor-1,3,5-s-triazin, Tris-(2,2,6,6-tetramethyl-4-piperidyl)-nitrilotriacetat, Tetrakis-(2,2,6,6-tetramethyl-4-piperidyl)-1,2,3,4-butantetraoat, 1,1'-(1,2-Ethandiyl)-bis-(3,3,5,5-tetramethyl-piperazinon), 4-Benzoyl-2,2,6,6-tetramethylpiperidin, 4-Stearyloxy-2,2,6,6-tetramethylpiperidin, Bis-(1,2,2,6,6-pentamethylpiperidyl)-2-n-butyl-2-(2-hydroxy-3,5-di-tert-butylbenzyl)-malonat, 3-n-Octyl-7,7,9,9-tetramethyl-1,3,8-triazaspiro[4.5]decan-2,4-dion, Bis-(1-octyloxy-2,2,6,6-tetramethylpiperidyl)-sebacat, Bis-(1-octyloxy-2,2,6,6-tetramethylpiperidyl)-succinat, Kondensationsprodukt aus N,N'-Bis-(2,2,6,6-tetramethyl-4-piperidyl)-hexamethylendiamin und 4-Morpholino-2,6-dichlor-1,3,5-triazin, Kondensationsprodukt aus 2-Chlor-4,6-di-(4-n-butylamino-2,2,6,6-tetramethylpiperidyl)-1,3,5-triazin und 1,2-Bis-(3-aminopropylamino)äthan, Kondensationsprodukt aus 2-Chlor-4,6-di-(4-n-butylamino-1,2,2,6,6-pentamethylpiperidyl)-1,3,5-triazin und 1,2-Bis-(3-aminopropylamino)-äthan, 8-Acetyl-3-dodecyl-7,7,9,9-tetramethyl-1,3,8-triazaspiro[4.5]decan-2,4-dion, 3-Dodecyl-1-(2,2,6,6-tetramethyl-4-piperidyl)pyrrolidin-2,5-dion, 3-Dodecyl-1-(1,2,2,6,6-pentamethyl-4-piperidyl)-pyrrolidin-2,5-dion.

2.7. Oxalsäurediamide, wie z.B. 4,4'-Di-octyloxy-oxanilid, 2,2'-Diethoxy-oxanilid, 2,2'-Di-octyloxy-5,5'-di-tert-butyl-oxanilid, 2,2'-Di-dodecyloxy-5,5'di-tert-butyl-oxanilid, 2-Ethoxy-2'-ethyl-oxanilid, N,N'-Bis-(3-dimethylaminopropyl)-oxalamid, 2-Ethoxy-5-tert-butyl-2'-ethyloxanilid und dessen Gemisch mit 2-Ethoxy-2'-ethyl-5,4'-di-tert-butyl-oxanilid, Gemische von o- und p-Methoxy- sowie von o- und p-Ethoxy-di-substituierten Oxaniliden.

2.8. 2-(2-Hydroxyphenyl)-1,3,5-triazine, wie z.B. 2,4,6-Tris(2-hydroxy-4-octyloxyphenyl)-1,3,5-triazin, 2-(2-Hydroxy4-octyloxyphenyl)-4,6-bis-(2,4-dimethylphenyl)-1,3,5-triazin, 2-(2,4-Dihydroxyphenyl)-4,6-bis(2,4-dimethylphenyl)-1,3,5-triazin, 2,4-Bis-(2-hydroxy-4-propyloxyphenyl)-6-(2,4-dimethylphenyl)-1,3,5-triazin, 2-(2-Hydroxy-4-octyloxyphenyl)-4,6-bis(4-methylphenyl)-1,3,5-triazin, 2-(2-Hydroxy-4-dodecyloxyphenyl)-4,6-bis(2,4-dimethylphenyl)-1,3,5-triazin, 2-[2-hydroxy-4-(2-hydroxy-3-butyloxy-propyloxy)phenyl]-4,6-bis(2,4-dimethylphenyl)-1,3,5-triazin, 2-[2-hydroxy-4-(2-hydroxy-3-octyloxy-propyloxy)phenyl]-4,6-bis(2,4-dimethylphenyl)-1,3,5-triazin.

3. Metalldesaktivatoren, wie z.B. N,N'-Diphenyloxalsäurediamid, N-Salicylal-N'-salicyloylhydrazin, N,N'-Bis-(salicyloyl)-hydrazin, N,N'-Bis-(3,5-di-tert-butyl-4-hydroxyphenylpropionyl)-hydrazin, 3-Salicyloylamino-1,2,4-triazol, Bis-(benzyliden)-oxalsäuredihydrazid, Oxanilid, Isophthalsäure-dihydrazid, Sebacinsäure-bis-phenylhydrazid, N,N'-Diacetyl-adipinsäure-dihydrazid, N,N'-Bis-salicyloyl-oxalsäure-dihydrazid, N,N'-Bis-salicyloyl-thiopropionsäure-dihydrazid.

4. Phosphite und Phosphonite, wie z.B. Triphenylphosphit, Diphenylalkylphosphite, Phenyldialkylphosphite, Tris-(nonylphenyl)-phosphit, Trilaurylphosphit, Trioctadecylphosphit, Distearyl-pentaerythritdiphosphit, Tris-(2,4-di-tert-butylphenyl)-phosphit, Diisodecylpentaerythrit-diphosphit, Bis-(2,4-di-tert-butylphenyl)-pentaerythritdiphosphit, Bis-(2,6-di-tert-butyl-4-methylphenyl)-pentaerythritdiphosphit, Bis-isodecyloxy-pentaerythritdiphosphit, Bis-(2,4-di-tert-butyl-6-methylphenyl)-pentaerythritdiphosphit, Bis-(2,4,6-tri-tert-butylphenyl)-pentaerythritdiphosphit, Tristearyl-sorbit-triphosphit, Tetrakis-(2,4-di-tert-butylphenyl)-4,4'-biphenylen-diphosphonit, 6-Isooctyloxy-2,4,8,10-tetra-tert-butyl-12H-dibenz[d,g]-1,3,2-dioxaphosphocin, 6-Fluor-2,4,8,10-tetra-tert-butyl-12-methyl-di-benz[d,g]-1,3,2-dioxaphosphocin, Bis-(2,4-di-tert-butyl-6-methylphenyl)-methylphosphit, Bis-(2,4-di-tert-butyl-6-methylphenyl)-ethylphosphit.

5. Peroxidzerstörende Verbindungen, wie z.B. Ester der β-Thio-dipropionsäure, beispielsweise der Lauryl-, Stearyl-, Myristyl- oder Tridecylester, Mercaptobenzimidazol, das Zinksalz des 2-Mercaptobenzimidazols, Zink-dibutyl-dithiocarbamat, Dioctadecyldisulfid, Pentaerythrit-tetrakis-(β-dodecylmercapto)-propionat.

6. Polyamidstabilisatoren, wie z.B. Kupfersalze in Kombination mit Jodiden und/oder Phosphorverbindungen und Salze des zweiwertigen Mangans.

7. Basische Co-Stabilisatoren, wie z.B. Melamin, Polyvinylpyrrolidon, Dicyandiamid, Triallylcyanurat, Harnstoff-Derivate, Hydrazin-Derivate, Amine, Polyamide, Polyurethane, Alkali- und Erdalkalisalze höherer Fettsäuren, beispielsweise Ca-Stearat, Zn-Stearat, Mg-Behenat, Mg-Stearat, Na-Ricinoleat, K-Palmitat, Antimonbrenzcatechinat oder Zinnbrenzcatechinat.

8. Nukleierungsmittel, wie z.B. 4-tert-Butylbenzoesäure, Adipinsäure, Diphenylessigsäure.

9. Füllstoffe und Verstärkungsmittel, wie z.B. Calciumcarbonat, Silikate, Glasfasern, Asbest, Talk, Kaolin,

Glimmer, Bariumsulfat, Metalloxide und -hydroxide, Ruß, Graphit.

10. Sonstige Zusätze, wie z.B. Weichmacher, Gleitmittel, Emulgatoren, Pigmente, Optische Aufheller, Flammschutzmittel, Antistatika, Treibmittel.

11. Benzofuranone bzw. Indolinone, wie z.B. in US-A-4 325 863, US-A-4 338 244, US-A-5 175 312, US-A-5 216 052, US-A-5 252 643, DE-A-4 316 611, DE-A-4 316 622, DE-A-4 316 876, EP-A-0 589 839 oder EP-A-0 591 102 beschrieben, oder 3-[4-(2-Acetoxyethoxy)phenyl]-5,7-di-tert-butyl-benzofuran-2-on, 5,7-Di-tert-butyl-3-[4-(2-stearoyloxyethoxy)phenyl]-benzofuran-2-on, 3,3'-Bis-[5,7-di-tert-butyl-3-(4-[2-hydroxyethoxy]-phenyl)-benzofuran-2-on], 5,7-Di-tert-butyl-3-(4-ethoxyphenyl)benzofuran-2-on, 3-(4-Acetoxy-3,5-dimethylphenyl)-5,7-di-tert-butyl-benzofuran-2-on, 3-(3,5-Dimethyl-4-pivaloyloxy-phenyl)-5,7-di-tert-butyl-benzofuran-2-on.

Handelt es sich bei den erfindungsgemäßen Zusammensetzungen um solche auf Basis von funktionellen Flüssigkeiten, insbesondere Schmierstoffen und Hydraulikflüssigkeiten bzw. Metallbearbeitungsflüssigkeiten, können diese ebenfalls weitere Additive enthalten, die zugegeben werden, um gewisse Gebrauchseigenschaften zu verbessern, wie z.B. weitere Antioxidantien, Metalldesaktivatoren, Rostinhibitoren, Viskositäts-Index-Verbesserer, Stockpunkterniedriger, Dispergiermittel/Tenside und Hochdruck- und Verschleißschutzadditive (Extreme Pressure/Anti Wear Additives). Beispiele hierfür sind:

Beispiele für phenolische Antioxidantien: Solche können den vorstehenden Punkten 1.1. bis 1.17 entnommen werden.

Beispiele für aminische Antioxidantien:

N,N'-Di-isopropyl-p-phenylendiamin, N,N'-Di-sec-butyl-p-phenylendiamin, N,N'-Bis(1,4-dimethyl-pentyl)-p-phenylendiamin, N,N'-Bis(1-ethyl-3-methyl-pentyl)-p-phenylendiamin, N,N'-Bis(1-methyl-heptyl)-p-phenylendiamin, N,N'-Dicyclohexyl-p-phenylendiamin, N,N'-Diphenyl-p-phenylendiamin, N,N'-Di-(naphthyl-2)-p-phenylendiamin, N-Isopropyl-N'-phenyl-p-phenylendiamin, N-(1,3-Dimethyl-butyl)-N'-phenyl-p-phenylendiamin, N-(1-Methyl-heptyl)-N'-phenyl-p-phenylendiamin, N-Cyclohexyl-N'-phenyl-p-phenylendiamin, 4-(p-Toluol-sulfonamido)-diphenylamin, N,N'-Dimethyl-N,N'-di-sec-butyl-p-phenylendiamin, Diphenylamin, N-Allyldiphenylamin, 4-Isopropoxy-diphenylamin, N-Phenyl-1-naphthylamin, N-Phenyl-2-naphthylamin, octyliertes Diphenylamin, z.B. p,p'-Di-tert-octyldiphenylamin, 4-n-Butylaminophenol, 4-Butyrylamino-phenol, 4-Nonanoylamino-phenol, 4-Dodecanoylamino-phenol, 4-Octadecanoylamino-phenol, Di-(4-methoxyphenyl)-amin, 2,6-Di-tert-butyl-4-dimethylamino-methyl-phenol, 2,4'-Diamino-diphenylmethan, 4,4'-Diamino-diphenylmethan, N,N,N',N'-Tetramethyl-4,4'-diamino-diphenylmethan, 1,2-Di-[(2-methyl-phenyl)-amino]ethan, 1,2-Di-(phenylamino)-propan, (o-Tolyl)-biguanid, Di-[4-(1',3'-dimethyl-butyl)-phenyl]amin, tert-octyliertes N-Phenyl-1-naphthylamin, Gemisch aus mono- und dialkylierten tert-Butyl/tert-Octyldiphenylaminen, Gemisch aus mono- und dialkylierten Isopropyl/Isohexyl-diphenylaminen, Gemische aus mono- und dialkylierten tert-Butyldiphenylaminen, 2,3-Dihydro-3,3-dimethyl-4H-1,4-benzothiazin, Phenothiazin, N-Allylphenothiazin, N,N,N',N'-Tetraphenyl-1,4-diaminobut-2-en, N,N-Bis-(2,2,6,6-tetramethyl-piperidin-4-yl-hexamethylendiamin, Bis-(2,2,6,6-tetramethylpiperidin-4-yl)-sebacat, 2,2,6,6-Tetramethylpiperidin-4-on, 2,2,6,6-Tetramethylpiperidin-4-ol.

Beispiele für weitere Antioxidantien:

Aliphatische oder aromatische Phosphite, Ester der Thiodipropionsäure oder der Thiodiessigsäure, oder Salze der Dithiocarbamid- oder Dithiophosphorsäure, 2,2,12,12-Tetramethyl-5,9-dihydroxy-3,7,11-trithiatridecan und 2,2,15,15-Tetramethyl-5,-12-dihydroxy-3,7,10,14-tetrathiahexadecan.

Beispiele für Metall-Desaktivatoren, z.B. für Kupfer, sind:

a) Benzotriazole und deren Derivate, z.B. 4- oder 5-Alkylbenzotriazole (z.B. Tolutriazol) und deren Derivate, 4,5,6,7-Tetrahydrobenzotriazol, 5,5'-Methylenbis-benzotriazol; Mannich-Basen von Benzotriazol oder Tolutriazol wie 1-[Di(2-ethylhexyl)-aminomethyl)-tolutriazol und 1- [Di(2-ethylhexyl)aminomethyl)-benzotriazol; Alkoxyalkylbenzotriazole wie 1-(Nonyloxymethyl)-benzotriazol, 1-(1-Butoxyethyl)-benzotriazol und 1-(1-Cyclohexyloxybutyl)-tolutriazol.

b) 1,2,4-Triazole und deren Derivate, z.B. 3-Alkyl (oder Aryl)- 1,2,4-Triazole, Mannich-Basen von 1,2,4-Triazolen wie 1-[Di(2-ethylhexyl)aminomethyl-1,2,4-triazol; Alkoxyalkyl-1,2,4-triazole wie 1-(1-Butoxyethyl-1,2,4-triazol; acylierte 3-Amino-1,2,4-triazole.

c) Imidazolderivate, z.B. 4,4'-Methylenbis(2-undecyl-5-methylimidazol, Bis[(N-methyl)imidazol-2-yl]carbinol-octylether.

d) Schwefelhaltige heterocyclische Verbindungen, z.B. 2-Mercaptobenzthiazol, 2,5-Di-mercapto-1,3,4-thiadiazol

und deren Derivate; 3,5-Bis[di(2-ethylhexyl)amino-methyl]-1,3,4-thiadiazolin-2-on.

e) Aminoverbindungen, z.B. Salicyliden-propylendiamin, Salicylaminoguanidin und deren Salze.

Beispiele für Rost-Inhibitoren sind:

a) Organische Säuren, ihre Ester, Metallsalze, Aminsalze und Anhydride, z.B. Alkyl-und Alkenylbernsteinsäuren und deren Partialester mit Alkoholen, Diolen oder Hydroxycarbonsäuren, Partialamide von Alkyl- und Alkenylbernsteinsäuren, 4-Nonylphenoxyessigsäure, Alkoxy- und Alkoxyethoxycarbonsäuren wie Dodecyloxyessigsäure, Dodecyloxy(ethoxy)-essigsäure und deren Aminsalze, ferner N-Oleoyl-sarcosin, Sorbitan-mono-oleat, Blei-naphthenat, Alkenylbernsteinsäureanhydride, z.B. Dodecenylbernsteinsäure-anhydrid, 2-Carboxymethyl-1-dodecyl-3-methylglycerin und dessen Aminsalze.

b) Stickstoffhaltige Verbindungen, z.B.:

i. Primäre, sekundäre oder tertiäre aliphatische oder cycloaliphatische Amine und Aminsalze von organischen und anorganischen Säuren, z.B. öllösliche Alkylammoniumcarboxylate, ferner 1-[N,N-bis-(2-hydroxyethyl)amino]-3-(4-nonylphenoxy)-propan-2-ol.

ii. Heterocyclische Verbindungen, z.B.:
Substituierte Imidazoline und Oxazoline, 2-Heptadecenyl-1-(2-hydroxyethyl)-imidazolin.

c) Phosphorhaltige Verbindungen, z.B.:
Aminsalze von Phosphorsäurepartialestern oder Phosphonsäurepartialestern, Zinkdialkyldithiophosphate.

d) Schwefelhaltige Verbindungen, z.B.:
Barium-dinonylnaphthalin-sulfonate, Calciumpetroleum-sulfonate, Alkylthio-substituierte aliphatische Carbonsäuren, Ester von aliphatischen 2-Sulfocarbonsäuren und deren Salze.

e) Glycerinderivate, z.B.:
Glycerin-monooleat, 1-(Alkylphenoxy)-3-(2-hydroxyethyl)glycerine, 1-(Alkylphenoxy)-3-(2,3-dihydroxypropyl)glycerine, 2-Carboxyalkyl-1,3-dialkylglycerine.

Beispiele für Viskositätsindex-Verbesserer sind:

Polyacrylate, Polymethacrylate, Vinylpyrrolidon/Methacrylat-Copolymere, Polyvinylpyrrolidone, Polybutene, Olefin-Copolymere, Styrol/Acrylat-Copolymere, Polyether.

Beispiele für Stockpunkterniedriger sind:

Polymethacrylat, alkylierte Naphthalinderivate.

Beispiele für Dispergiermittel/Tenside sind:

Polybutenylbernsteinsäureamide oder -imide, Polybutenylphosphonsäurederivate, basische Magnesium-, Calcium-, und Bariumsulfonate und -phenolate.

Beispiele für Hochdruck- und Verschleißschutz-Additive sind:

Schwefel und/oder Phosphor und/oder Halogen enthaltende Verbindungen, wie geschwefelte Olefine und pflanzliche Öle, Zinkdialkyldithiophosphate, alkylierte Triphenylphosphate, Tritolylphosphat, Tricresylphosphat, chlorierte Paraffine, Alkyl- und Aryldi-und tri-sulfide, Aminsalze von Mono- und Dialkylphosphaten, Aminsalze der Methylphosphonsäure, Diethanolaminomethyltolyltriazol, Di(2-ethylhexyl)aminomethyltolyltriazol, Derivate des 2,5-Dimercapto-1,3,4-thiadiazols, 3-[(Bis-isopropyloxy-phosphinothioyl)-thio]-propionsäure-ethylester, Triphenylthiophosphat (Triphenylphosphorothioat), Tris-(alkylphenyl)phosphorothioate und deren Gemische, (z.B. Tris(isononylphenyl)phosphorothioat), Diphenyl-monononylphenyl-phosphorothioat, Isobutylphenyl-diphenylphosphorothioat, Dodecylaminsalz des 3-Hydroxy-1,3-thiaphosphetan-3-oxids, Trithiophosphorsäure-5,5,5-tris[isooctylacetat (2)], Derivate von 2-Mercaptobenzthiazol wie 1-[N,N-Bis(2-ethylhexyl)aminomethyl-2-mercapto-1H-1,3-benzothiazol, Ethoxycarbonyl-5-octyl-dithiocarbamat.

Die vorliegende Erfindung betrifft auch die Verwendung von erfindungsgemäßen Reaktionsprodukten zum Stabilisieren von gegen oxidativen, thermischen und/oder lichtinduzierten Abbau empfindlichem organischem Material,

insbesondere von natürlichen oder (halb)synthetischen Polymeren oder von funktionellen Flüssigkeiten, vor allem von Schmierstoffen. Die Verbindungen wirken beispielsweise besonders gut als Antioxidantien und Ablagerungsverhinderer (deposit control agents) in funktionellen Flüssigkeiten, wie oben erwähnt.

Bevorzugte erfindungsgemäße Reaktionsprodukte, wie vorstehend beschrieben, führen zu bevorzugten Zusammensetzungen.

Die Erfindung betrifft auch ein Verfahren zur Herstellung von Stoffgemischen, das sich der anfangs beschriebenen Reaktion bedient. Die eingesetzten Ausgangsstoffe sind kommerziell erhältlich oder nach bekannten Verfahren herstellbar.

Die folgenden Beispiele erläutern die Erfindung weiter, ohne sie jedoch zu beschränken. Teile- und Prozentangaben beziehen sich, sofern nicht anders angegeben, auf das Gewicht.

<u>Beispiel 1</u>: In einem Dreihalskolben mit Rückflußkühler, Thermometer, Magnetrührer und Wasserabscheider werden 148.1 g (0.875 mol) Diphenylamin, 24.9 g (0.125 mol) Phenothiazin und 17.6 g Fulcat 22B® bei 100°C in 76.2 g (0.679 mol) Diisobutylen im leichten Stickstoffstrom zum Schmelzen gebracht. Innerhalb einer Stunde wird auf 175 °C erhitzt (starker Rückfluß), und währenddessen werden ca. 2 ml Wasser abgeschieden. Weitere 100 g (0.891 mol) Diisobutylen werden während ca. 4 h so zugetropft, daß die Innentemperatur bei 165-175°C liegt. Es wird bei 165-175°C ca. 5 h weitergerührt, bis die Diphenylaminkonzentration unter ca. 4% des Anfangswertes abgesunken ist.

Dann läßt man auf ca. 90°C abkühlen und filtriert den Katalysator ab. Das überschüssige Diisobutylen (ca. 50 g) wird bei 80°C während 2 Stunden bei vermindertem Druck (ca. 0.01 Torr) abdestilliert.

Man erhält 285 g eines leichtflüssigen, kaum gefärbten Öls.

Analyse: gefunden 84.22% C, 9.8 % H, 4.58% N und 1.36% S

<u>Beispiel 2</u>: In einem Dreihalskolben mit Rückflußkühler, Thermometer, Magnetrührer und Wasserabscheider werden 160.8 g (0.950 mol) Diphenylamin, 10.0 g (0.050 mol) Phenothiazin und 17.0 g Fulcat 22B® bei 100°C in 76.2 g (0.679 mol) Diisobutylen im leichten Stickstoffstrom zum Schmelzen gebracht. Innerhalb einer Stunde wird auf 175 °C erhitzt (starker Rückfluß) und währenddessen ca. 2 ml Wasser abgeschieden. Weitere 100 g (0.891 mol) Diisobutylen werden während ca. 3 h so zugetropft, daß die Innentemperatur bei 165-175°C liegt. Es wird bei 165-175°C ca. 3 h weitergerührt, bis die Diphenylaminkonzentration unter ca. 4% des Anfangswertes abgesunken ist.

Dann läßt man auf ca. 90°C abkühlen und filtriert den Katalysator ab. Das überschüssige Diisobutylen (ca. 34 g) wird bei 80°C während 2 Stunden bei vermindertem Druck (ca. 0.01 Torr) abdestilliert.

Man erhält 274 g eines leichtflüssigen, kaum gefärbten Öls.

Analyse: gefunden 84.99% C, 9.9 % H, 4.96% N und 0.55% S

<u>Beispiel 3</u>: In einem Dreihalskolben mit Rückflußkühler, Thermometer, Magnetrührer und Wasserabscheider werden 135.4 g (0.80 mol) Diphenylamin, 40.0 g (0.20 mol) Phenothiazin und 17.0 g Fulcat 22B® bei 100°C in 76.2 g (0.679 mol) Diisobutylen im leichten Stickstoffstrom zum Schmelzen gebracht. Innerhalb einer Stunde wird auf 175 °C erhitzt (starker Rückfluß) und währenddessen ca. 2 ml Wasser abgeschieden. Weitere 100 g (0.891 mol) Diisobutylen werden während ca. 3 h so zugetropft, daß die Innentemperatur bei 165-175°C liegt. Es wird bei 165-175°C ca. 4 h weitergerührt, bis die Diphenylaminkonzentration unter ca. 4% des Anfangswertes abgesunken ist.

Dann läßt man auf ca. 90°C abkühlen und filtriert den Katalysator ab. Das überschüssige Diisobutylen (ca. 28 g) wird bei 80°C während 2 Stunden bei vermindertem Druck (ca. 0.01 Torr) abdestilliert.

Man erhält 291 g eines leichtflüssigen, kaum gefärbten Öls.

Analyse: gefunden 83.85% C, 9.74 % H, 4.43% N und 2.11% S

<u>Beispiel</u> 4: In einem Dreihalskolben mit Rückflußkühler, Thermometer, Magnetrührer und Wasserabscheider werden 145.8 g (0.861 mol) Diphenylamin, 27.6 g (0.139 mol) Phenothiazin und 17.0 g Fulcat 22B® bei 100°C in 76.2 g (0.679 mol) Diisobutylen im leichten Stickstoffstrom zum Schmelzen gebracht. Innerhalb einer Stunde wird auf 175 °C erhitzt (starker Rückfluß) und währenddessen ca. 2 ml Wasser abgeschieden. Weitere 100 g (0.891 mol) Diisobutylen werden während ca. 3 h so zugetropft, daß die Innentemperatur bei 165-175°C liegt. Es wird bei 165-175°C ca. 4 h weitergerührt, bis die Diphenylaminkonzentration unter ca. 4% des Anfangswertes abgesunken ist.

Dann läßt man auf ca. 90°C abkühlen und filtriert den Katalysator ab. Das überschüssige Diisobutylen (ca. 27 g) wird bei 80°C während 2 Stunden bei vermindertem Druck (ca. 0.01 Torr) abdestilliert.

Man erhält 295 g eines leichtflüssigen, kaum gefärbten Öls.

Analyse: gefunden 84.39% C, 9.86 % H, 4.73% N und 1.49% S

Beispiel 5: In einem Dreihalskolben mit Rückflußkühler, Thermometer, Magnetrührer und Wasserabscheider werden unter Stickstoff 67.6 g (0.4 mol) Diphenylamin, 20.1 g (0.1 mol) Phenothiazin und 8.8 g Fulcat 22B® bei 100°C (Heizbad) in 40 ml Toluol vorgelegt. Innerhalb einer Stunde wird auf 165 °C erhitzt. Währenddessen scheiden sich ca. 1 ml Wasser ab. 168.3 g (1.0 mol) 1-Dodecen werden während ca. 1 h so zugetropft, daß die Innentemperatur nicht unter 165°C sinkt liegt. Es wird bei 165-175°C ca. 12 h weitergerührt, bis die Diphenylaminkonzentration unter ca. 4% des Anfangswertes abgesunken ist.

Dann läßt man auf ca. 90°C abkühlen und filtriert den Katalysator ab. Das überschüssige 1-Dodecen wird bei 80°C während 2 Stunden bei vermindertem Druck (ca. 0.01 Torr) abdestilliert.

Man erhält 199 g eines braun gefärbten Öls.

Analyse: gefunden 83.94% C, 11.26 % H, 3.29% N und 1.57% S
Gaschromatographie (GC): DPA-Gehalt im Produkt: -

Beispiel 6: In einem Dreihalskolben mit Rückflußkühler, Thermometer, Magnetrührer und Wasserabscheider werden 87.7 g (0.4 mol) Phenyl-$\alpha$-naphthylamin (PANA) 20.1 g (0.1 mol) Phenothiazin und 8.8 g Fulcat 22B® unter Stickstoff bei 100°C (Heizbad) in 40 ml Toluol vorgelegt. Innerhalb einer Stunde wird auf 165 °C erhitzt. Währenddessen scheiden sich ca. 1 ml Wasser ab. 168 g (1.0 mol) 1-Dodecen werden während ca. 1 h so zugetropft, daß die Innentemperatur nicht unter 165°C liegt. Es wird bei 165-175°C ca. 12 h weitergerührt, bis die PANA-Konzentration unter ca. 4% des Anfangswertes abgesunken ist.

Dann läßt man auf ca. 90°C abkühlen und filtriert den Katalysator ab. Das überschüssige 1-Dodecen wird bei 80°C während 2 Stunden bei vermindertem Druck (ca. 0.01 Torr) abdestilliert.

Man erhält 189 g eines braun gefärbten Öls.

Analyse: gefunden 85.10% C, 9.97 % H, 3.21% N und 1.51% S
GC: PANA-Gehalt im Produkt: um 1%

Beispiel 7: In einem Dreihalskolben mit Rückflußkühler, Thermometer, Magnetrührer und Wasserabscheider werden 16.9 g (0.1 mol) Diphenylamin, 39.9 g (0.1 mol) Phenothiazin und 6 g Fulcat 22B® bei 100°C (Heizbad) in 40 ml Toluol vorgelegt. Innerhalb einer Stunde wird auf 165 °C erhitzt. Währenddessen scheiden sich ca. 1/2 ml Wasser ab. 75.8 g (0.6 mol) Tripropylen werden während ca. 1 h so zugetropft, daß die Innentemperatur nicht unter 165°C liegt. Es wird bei 165-175°C ca. 5 h weitergerührt, bis die Diphenylaminkonzentration unter ca. 4% des Anfangswertes abgesunken ist.

Dann läßt man auf ca. 90°C abkühlen und filtriert den Katalysator ab. Das überschüssige Tripropylen wird bei 80°C während 2 Stunden bei vermindertem Druck (ca. 0.01 Torr) abdestilliert.

Man erhält 94 g eines braun gefärbten Öls.

Analyse: gefunden 80.30% C, 9.30 % H, 4.30% N und 6.14% S
GC: DPA-Gehalt: 3.2%; Phenothiazingehalt: 4.1%.

Beispiel 8: Deposit and Oxidation Panel Test (DOPT)

Es handelt sich bei diesem Test um eine Variante einer Prüfmethode für Motoröle, insbesondere für Dieselmotoröle, welche von G. Abellaneda et al., IIIe Symposium CEC, 1989, 61 New Cavendish Street, London WIM8AR, England, beschrieben wurde. Er dient dazu, die Eignung der Öle mit dem jeweiligen Stabilisator zur Verhinderung von Ablagerungen am Kolben zu prüfen.

Dabei wird in oxidierender Atmosphäre mit definierter Geschwindigkeit Öl auf eine saubere, vorher gewogene, schrägstehende heiße Metallplatte (Panel) getropft und so ein Ölfilm erzeugt. Die Testdauer beträgt 20 Stunden, die Temperatur auf der Metallplatte 260°C, der Luftdurchsatz 9.7 l/h und die Fließgeschwindigkeit des Öls 1 ml/min. Die feuchte Luft-Atmosphäre wird mit 460 ppm $NO_2$ und 25 ppm $SO_2$ angereichert.

Nach dem Test wird die Metallplatte zur Entfernung des Öls in Petrolether getaucht, getrocknet, gewogen und visuell bewertet, z.B. daraufhin, ob sich ein Lack gebildet hat, und wie der Rückstand gefärbt ist.
Je kleiner das Gewicht des Rückstandes und die visuelle Bewertungszahl sind, desto besser ist das Ergebnis.
Als Schmieröl wird handelsübliches Low Reference CCMC Diesel Engine Oil verwendet.

Diesem vorbereiteten Öl werden die in Tabelle I angegebenen Stabilisatoren in einer Menge von 0.6 Gew.%, bezogen auf das Öl, zugemischt und der DOPT Prüfung unterzogen.

Tabelle I

| Deposit and Oxidation Panel Test | | | |
|---|---|---|---|
| Produkt gemäß Beispiel | Konzentration [Gew %] | Ablagerung | |
| | | Gewicht [mg] | visuell |
| 3 | 0.6 | 21 | 10 |
| 4 | 0.6 | 22 | 10 |
| ohne Zusatz | - | 145 | 15 |

**Patentansprüche**

1. Reaktionsprodukt, erhältlich aus der Umsetzung eines Olefins der Formel

$$R_1R_2C{=}CHR_3 \qquad (I)$$

mit
einer Mischung von Verbindungen der Formeln II und III

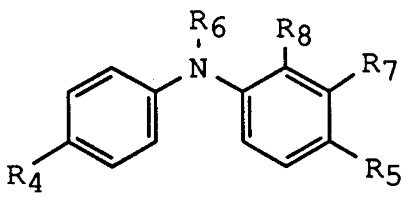 (II) 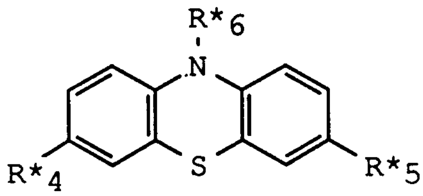 (III)

in Gegenwart eines sauren Katalysators,

wobei $R_1$ Wasserstoff, $C_1$-$C_{25}$-Alkyl oder Phenyl,
$R_2$ $C_1$-$C_{25}$-Alkyl, Benzyl oder Phenyl,
$R_3$ Wasserstoff oder $C_1$-$C_{12}$-Alkyl,
$R_4$, $R^*_4$, $R_5$ und $R^*_5$ unabhängig voneinander Wasserstoff, $C_1$-$C_{25}$-Alkyl, $C_5$-$C_{12}$-Cycloalkyl, Benzyl, $\alpha$-Methylbenzyl oder $\alpha,\alpha$-Dimethylbenzyl, und
$R_6$ und $R^*_6$ unabhängig voneinander Wasserstoff, $C_1$-$C_{18}$-Alkyl, $C_3$-$C_4$-Alkenyl, Benzyl oder ein Rest der Formel $-CH(R_9)-CH(R_{10})-S-R_{11}$ sind, sowie
$R_7$ und $R_8$ Wasserstoff bedeuten oder zusammen eine zweiwertige Gruppe

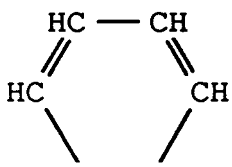

bilden,
$R_9$ und $R_{10}$ unabhängig voneinander Wasserstoff, Phenyl oder $C_1$-$C_{12}$-Alkyl sind,
$R_{11}$ $C_4$-$C_{18}$-Alkyl, Phenyl oder $-(CH_2)_a-CO-OR_{12}$ bedeutet,
a 1 oder 2 ist, und
$R_{12}$ für $C_1$-$C_{18}$-Alkyl steht.

2. Produkt nach Anspruch **1**, wobei

$R_1$ Wasserstoff oder $C_1$-$C_{12}$-Alkyl,

$R_2$ $C_1$-$C_{18}$-Alkyl oder Phenyl,
$R_3$ Wasserstoff oder $C_1$-$C_6$-Alkyl,
$R_4$, $R^*_4$, $R_5$ und $R^*_5$ Wasserstoff, $C_4$-$C_{18}$-Alkyl, Cyclohexyl, Benzyl, α-Methylbenzyl oder α,α-Dimethylbenzyl und
$R_6$ Wasserstoff, Methyl, Allyl oder Benzyl
sind.

**3.** Produkt nach Anspruch **1**, wobei

$R_1$ Wasserstoff oder $C_1$-$C_4$-Alkyl,
$R_2$ $C_1$-$C_{18}$-Alkyl und
$R_3$ Wasserstoff oder $C_1$-$C_4$-Alkyl sind.

**4.** Produkt nach Anspruch **1**, wobei

$R_1$ und $R_3$ Wasserstoff oder Methyl und
$R_2$ $C_1$-$C_{12}$-Alkyl sind.

**5.** Produkt nach Anspruch **1**, wobei

$R_4$, $R^*_4$, $R_5$, und $R^*_5$ Wasserstoff sind.

**6.** Produkt nach Anspruch **1**, wobei das Molverhältnis Verbindung der Formel II : Verbindung der Formel III 5 : 95 bis 95 : 5 ist.

**7.** Produkt nach Anspruch **6**, wobei das Molverhältnis 70 : 30 bis 30 : 70 ist.

**8.** Produkt nach Anspruch **6**, wobei das Molverhältnis 70 : 30 bis 95 : 5 ist.

**9.** Produkt nach Anspruch **6**, wobei das Molverhältnis 80 : 20 bis 90 : 10 ist.

**10.** Produkt nach Anspruch **1**, wobei die Verbindung der Formel I in einer Menge von 0.5 bis 4 Mol pro Mol der Mischung aus den Verbindungen der Formeln II und III eingesetzt wird.

**11.** Produkt nach Anspruch **10**, wobei die Verbindung der Formel I in einer Menge von 1 bis 2 Mol pro Mol der Mischung aus den Verbindungen der Formeln II und III eingesetzt wird.

**12.** Produkt nach Anspruch **1**, wobei der Katalysator der Gruppe der Brønsted-Säuren, Lewis-Säuren, Aluminiumsilikate, Ionenaustauscherharze, Zeolithe, natürlich vorkommenden Schichtsilikate oder modifizierten Schichtsilikate entstammt.

**13.** Produkt nach Anspruch **12**, wobei die Brønsted- oder Lewis-Säure in einer Menge von 0.002-10 Mol%, bezogen auf die Mischung aus den Verbindungen der Formeln II und III eingesetzt wird, und die Aluminiumsilikate, Ionenaustauscherharze, Zeolithe, natürlich vorkommenden Schichtsilikate oder modifizierten Schichtsilikate in einer Menge von 1-50 Gewichts%, bezogen auf die Mischung aus den Verbindungen der Formeln II und III eingesetzt werden.

**14.** Produkt nach Anspruch **1**, wobei der Katalysator ein natürlich vorkommendes Schichtsilikat ("saure Erde") vom Typ der Bentonite oder Montmorillonite ist.

**15.** Produkt nach Anspruch **14**, wobei 5-20 Gewichts% Katalysator eingesetzt werden.

**16.** Zusammensetzung enthaltend

A) ein gegen lichtinduzierten, thermischen oder /und oxidativen Abbau empfindliches organisches Material und
B) mindestens ein Produkt gemäß Anspruch **1**.

**17.** Zusammensetzung nach Anspruch **16**, worin das organische Material ein natürliches, halbsynthetisches oder synthetisches Polymer oder eine funktionelle Flüssigkeit ist.

**18.** Zusammensetzung nach Anspruch **17**, worin das organische Material ein Schmierstoff oder eine Metallbearbeitungs- oder Hydraulikflüssigkeit ist.

**19.** Verwendung von Produkten gemäß Anspruch **1** zum Stabilisieren von gegen oxidativen, thermischen und/oder lichtinduzierten Abbau empfindlichem organischen Material.

**20.** Verwendung nach Anspruch **19** zum Stabilisieren von natürlichen, halbsynthetischen oder synthetischen Polymeren oder von funktionellen Flüssigkeiten.

**21.** Verwendung nach Anspruch **19** zum Stabilisieren von Schmierstoffen, Metallbearbeitungs- oder Hydraulikflüssigkeiten.

**22.** Verfahren zum Stabilisieren von organischem Material gegen oxidativen, thermischen und/oder lichtinduzierten Abbau, dadurch gekennzeichnet, daß man diesem Material als Stabilisator mindestens ein Produkt gemäß Anspruch **1** zusetzt bzw. auf dieses aufbringt.

**23.** Verfahren nach Anspruch **22** zum Stabilisieren von natürlichen, halbsynthetischen oder synthetischen Polymeren oder von funktionellen Flüssigkeiten.

**24.** Verfahren nach Anspruch **23** zum Stabilisieren von synthetischen Polymeren oder von Schmierstoffen, Metallbearbeitungs- oder Hydraulikflüssigkeiten.

**25.** Verfahren zur Herstellung eines Stoffgemisches, dadurch gekennzeichnet, daß ein Olefin der Formel

$$R_1R_2C{=}CHR_3 \qquad (I)$$

mit
einer Mischung von Verbindungen der Formeln II und III

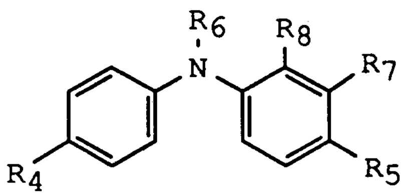
(II)

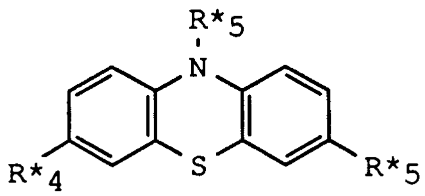
(III)

in Gegenwart eines sauren Katalysators umgesetzt wird,

wobei $R_1$ Wasserstoff, $C_1$-$C_{25}$-Alkyl oder Phenyl,
$R_2$ $C_1$-$C_{25}$-Alkyl, Benzyl oder Phenyl, $R_3$ Wasserstoff oder $C_1$-$C_{12}$-Alkyl,
$R_4$, $R^*_4$, $R_5$ und $R^*_5$ unabhängig voneinander Wasserstoff, $C_1$-$C_{25}$-Alkyl, $C_5$-$C_{12}$-Cycloalkyl, Benzyl, α-Methylbenzyl oder α,α-Dimethylbenzyl, und
$R_6$ und $R^*_6$ unabhängig voneinander Wasserstoff, $C_1$-$C_{18}$-Alkyl, $C_3$-$C_4$-Alkenyl, Benzyl oder ein Rest der Formel -CH($R_9$)-CH($R_{10}$)-S-$R_{11}$ sind, sowie
$R_7$ und $R_8$ Wasserstoff bedeuten oder zusammen eine zweiwertige Gruppe

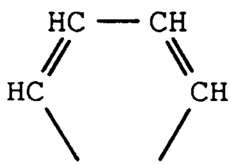

bilden,

$R_9$ und $R_{10}$ unabhängig voneinander Wasserstoff, Phenyl oder $C_1$-$C_{12}$-Alkyl sind,
$R_{11}$ $C_4$-$C_{18}$-Alkyl, Phenyl oder -$(CH_2)_a$-CO-$OR_{12}$ bedeutet,
a 1 oder 2 ist, und
$R_{12}$ für $C_1$-$C_{18}$-Alkyl steht.

## Claims

1. A reaction product obtainable by the reaction of an olefin of formula

$$R_1R_2C=CHR_3 \quad (I)$$

with
a mixture of compounds of formulae II and III

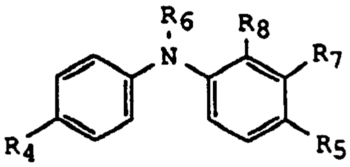
(II)
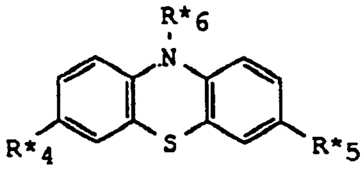
(III)

in the presence of an acid catalyst,

wherein $R_1$ is hydrogen, $C_1$-$C_{25}$alkyl or phenyl,
$R_2$ is $C_1$-$C_{25}$alkyl, benzyl or phenyl,
$R_3$ is hydrogen or $C_1$-$C_{12}$alkyl,
$R_4$, $R^*_4$, $R_5$ and $R^*_5$ are each independently of one another hydrogen, $C_1$-$C_{25}$alkyl, $C_5$-$C_{12}$cycloalkyl, benzyl, α-methylbenzyl or α,α-dimethylbenzyl, and
$R_6$ and $R^*_6$ are each independently of the other, $C_1$-$C_{18}$alkyl, $C_3$-$C_4$alkenyl, benzyl or a radical of formula -CH($R_9$) -CH($R_{10}$) -$SR_{11}$, and
$R_7$ and $R_8$ are hydrogen, or together form a divalent group

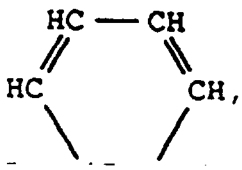

$R_9$ and $R_{10}$ are each independently of the other hydrogen, phenyl or $C_1$-$C_{12}$alkyl,
$R_{11}$ is $C_4$-$C_{18}$alkyl, phenyl or -$(CH_2)_a$-CO-$OR_{12}$,
a is 1 or 2, and
$R_{12}$ is $C_1$-$C_{18}$alkyl.

2. A product according to claim **1**, wherein

$R_1$ is hydrogen or $C_1$-$C_{12}$alkyl,
$R_2$ is $C_1$-$C_{18}$alkyl or phenyl,
$R_3$ is hydrogen or $C_1$-$C_6$alkyl,
$R_4$, $R^*_4$, $R_5$ and $R^*_5$ are hydrogen, $C_4$-$C_{18}$alkyl, cyclohexyl, benzyl, α-methylbenzyl or α,α-dimethylbenzyl, and
$R_6$ is hydrogen, methyl, allyl or benzyl.

3. A product according to claim **1**, wherein

$R_1$ is hydrogen or $C_1$-$C_4$alkyl,

$R_2$ is $C_1$-$C_{18}$alkyl, and
$R_3$ is hydrogen or $C_1$-$C_4$alkyl.

**4.** A product according to claim **1**, wherein

$R_1$ and $R_3$ are hydrogen or methyl, and
$R_2$ is $C_1$-$C_{12}$alkyl.

**5.** A product according to claim **1**, wherein

$R_4$, $R^*_4$, $R_5$ and $R^*_5$ are hydrogen.

**6.** A product according to claim **1**, wherein the molar ratio of compound of formula II:compound of formula III is from 5:95 to 95:5.

**7.** A product according to claim **6**, wherein the molar ratio is from 70:30 to 30:70.

**8.** A product according to claim **6**, wherein the molar ratio is from 70:30 to 95:5.

**9.** A product according to claim **6**, wherein the molar ratio is from 80:20 to 90:10.

**10.** A product according to claim **1**, wherein the compound of formula I is used in an amount of 0.5 to 4 mol per mole of the mixture of the compounds of formulae II and III.

**11.** A product according to claim **10**, wherein the compound of formula I is used in an amount of 1 to 2 mol per mole of the mixture of the compounds of formulae II and III.

**12.** A product according to claim **1**, wherein the catalyst is from the group of the Brønsted acids, Lewis acids, aluminium silicates, ion exchange resins, zeolites, naturally occuring sheet silicates or modified sheet silicates.

**13.** A product according to claim **12**, wherein the Brønsted or Lewis acid is used in an amount of 0.002-10 mol%, based on the mixture of the compounds of formulae II and III, and the aluminium silicates, ion exchange resins, zeolites, naturally occuring sheet silicates or modified sheet silicates are used in an amount of 1-50% by weight, based of the mixture of the compounds of formulae II and III.

**14.** A product according to claim **1**, wherein the catalyst is a naturally occuring sheet silicate ("acid clay") of the bentonite or montmorillonite type.

**15.** A product according to claim **14**, wherein 5-20% by weight of catalyst is used.

**16.** A composition comprising

A) an organic material susceptible to light-induced, thermal and/or oxidative degradation, and
B) at least one product according to claim **1**.

**17.** A composition according to claim **16**, wherein the organic material is a natural, semi-synthetic or synthetic polymer or a functional fluid.

**18.** A composition according to claim **17**, wherein the organic material is a lubricant or a metalworking fluid or hydraulic fluid.

**19.** Use of a product according to claim **1** for stablising organic material susceptible to oxidative, thermal and/or light-induced degradation.

**20.** Use according to claim **19** for stablising natural, semi-synthetic or synthetic polymers or functional fluids.

**21.** Use according to claim **19** for stabilising lubricants, metalworking fluids or hydraulic fluids.

**22.** A process for stablising organic material against oxidative, thermal and/or light-induced degradation, which comprises adding or applying to said material at least one product according to claim **1** as stabiliser.

**23.** A process according to claim **22** for stabilising natural, semi-synthetic or synthetic polymers or functional fluids.

**24.** A process according to claim **23** for stabilising synthetic polymers or lubricants, metalworking fluids or hydraulic fluids.

**25.** A process for the preparation of a composition of matter, which comprises reacting an olefin of formula

$$R_1R_2C{=}CHR_3 \quad (I)$$

with
a mixture of compounds of formulae II and III

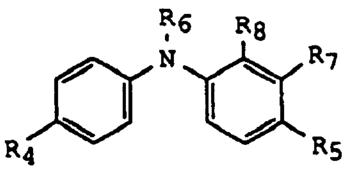 (II) 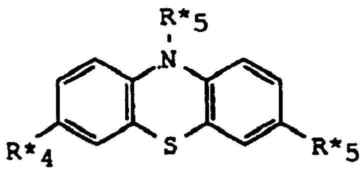 (III)

in the presence of an acid catalyst,

wherein $R_1$ is hydrogen, $C_1$-$C_{25}$alkyl or phenyl,
$R_2$ is $C_1$-$C_{25}$alkyl, benzyl or phenyl,
$R_3$ is hydrogen or $C_1$-$C_{12}$alkyl,
$R_4$, $R^*_4$, $R_5$ and $R^*_5$ are each independently of one another hydrogen, $C_1$-$C_{25}$alkyl, $C_5$-$C_{12}$cycloalkyl, benzyl, $\alpha$-methylbenzyl or $\alpha,\alpha$-dimethylbenzyl, and
$R_6$ and $R^*_6$ are each independently of the other, $C_1$-$C_{18}$alkyl, $C_3$-$C_4$alkenyl, benzyl or a radical of formula -$CH(R_9)$ -$CH$ $(R_{10})$ -S-$R_{11}$, and
$R_7$ and $R_8$ are hydrogen, or together form a divalent group

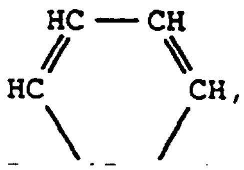

$R_9$ and $R_{10}$ are each independently of the other hydrogen, phenyl or $C_1$-$C_{12}$alkyl,
$R_{11}$ is $C_4$-$C_{18}$alkyl, phenyl or - $(CH_2)_a$-CO-O$R_{12}$,
a is 1 or 2, and
$R_{12}$ is $C_1$-$C_{18}$alkyl.

**Revendications**

**1.** Produit réactionnel obtenu par action d'une oléfine de formule

$$R_1R_2C{=}CHR_3 \quad (I)$$

sur un mélange de composés de formules II et III

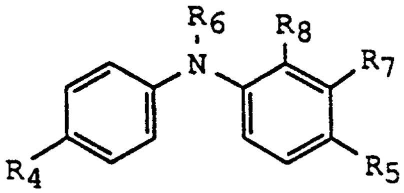
(II)
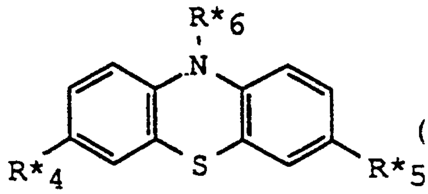
(III)

en présence d'un catalyseur acide,
où

$R_1$ représente un atome d'hydrogène, des groupes alkyle en $C_1$-$C_{25}$ ou phényle,
$R_2$ représente des groupes alkyle en $C_1$-$C_{25}$, benzyle ou phényle,
$R_3$ représente un atome d'hydrogène ou un groupe alkyle en $C_1$-$C_{12}$,
$R_4$, $R^*_4$, $R_5$ et $4^*_5$ représentent, indépendamment les uns des autres, des atomes d'hydrogène, des groupes alkyle en $C_1$-$C_{25}$, cycloalkyle en $C_5$-$C_{12}$, benzyle, α-méthylbenzyle ou α,α-diméthylbenzyle, et
$R_6$ et $R^*_6$ représentent, indépendamment l'un de l'autre, des atomes d'hydrogène, des groupes alkyle en $C_1$-$C_{18}$, alcényle en $C_3$-$C_4$, benzyle ou un reste de formule $-CH(R_9)-CH(R_{10})-S-R_{11}$, ainsi que
$R_7$ et $R_8$ représentent des atomes d'hydrogène ou ensemble forment un groupe bivalent

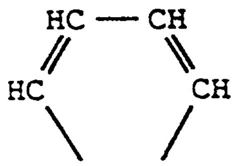

$R_9$ et $R_{10}$ représentent, indépendamment l'un de l'autre, des atomes d'hydrogène, des groupes phényle ou alkyle en $C_1$-$C_{12}$,
$R_{11}$ représente des groupes alkyle en $C_4$-$C_{18}$, phényle ou $-(CH_2)_a-CO-OR_{12}$,
a vaut 1 ou 2, et
$R_{12}$ représente un groupe alkyle en $C_1$-$C_{18}$.

**2.** Produit selon la revendication 1, où

$R_1$ représente un atome d'hydrogène ou un groupe alkyle en $C_1$-$C_{12}$,
$R_2$ représente des groupes alkyle en $C_1$-$C_{18}$ ou phényle,
$R_3$ représente un atome d'hydrogène ou un groupe alkyle en $C_1$-$C_6$,
$R_4$, $R^*_4$, $R_5$ et $R^*_5$ représentent des atomes d'hydrogène, des groupes alkyle en $C_4$-$C_{18}$, cyclohexyle, benzyle, α-méthylbenzyle ou α,α-diméthylbenzyle, et
$R_6$ représente un atome d'hydrogène, des groupes méthyle, allyle ou benzyle.

**3.** Produit selon la revendication 1, où

$R_1$ représente un atome d'hydrogène ou un groupe alkyle en $C_1$-$C_4$,
$R_2$ représente un groupe alkyle en $C_1$-$C_{18}$ et
$R_3$ représente un atome d'hydrogène ou un groupe alkyle en $C_1$-$C_4$.

**4.** Produit selon la revendication 1, où

$R_1$ et $R_3$ représentent un atome d'hydrogène ou un groupe méthyle et
$R_2$ représente un groupe alkyle en $C_1$-$C_{12}$.

**5.** Produit selon la revendication 1, où

$R_4$, $R^*_4$, $R_5$ et $R^*_5$ représentent des atomes d'hydrogène.

6. Produit selon la revendication 1, où le rapport molaire de composé de formule II:composé de formule III est de 5:95 à 95:5.

7. Produit selon la revendication 1, où le rapport molaire est de 70:30 à 30:70.

8. Produit selon la revendication 6, où le rapport molaire est de 70:30 à 95:5.

9. Produit selon la revendication 6, où le rapport molaire est de 80:20 à 90:10.

10. Produit selon la revendication 1, où le composé de formule I est utilisé en une quantité de 0,5 à 4 moles par mole du mélange des composés de formules II et III.

11. Produit selon la revendication 10, où le composé de formule I est utilisé en une quantité de 1 à 2 moles par mole du mélange des composés de formules II et III.

12. Produit selon la revendication 1, où le catalyseur est pris dans le groupe des acides de Brønsted, des acides de Lewis, des aluminosilicates, des résines d'échangeuses d'ions, des zéolithes, des phyllosilicates naturels ou phyllosilicates modifiés.

13. Produit selon la revendication 12, où l'acide de Brønsted ou de Lewis est utilisé en une quantité de 0,002 à 10 % molaires, par rapport au mélange des composés de formules II et III, et les aluminosilicates, les résines échangeuses d'ions, les zéolites, les phyllosilicates naturels ou les phyllosilicates modifiés en une quantité de 1 à 50 % massiques, par rapport au mélange des composés de formules II et III.

14. Produit selon la revendication 1, où le catalyseur est un phyllosilicate naturel ("terre acide") du type bentonites ou montmorillonites.

15. Produit selon la revendication 14, où l'on utilise 5 à 20 % massiques de catalyseur.

16. Composition contenant

    A) une matière organique sensible à la dégradation induite par la lumière, thermique et/ou oxydative et
    B) au moins un produit selon la revendication 1.

17. Composition selon la revendication 16, où la matière organique est un polymère naturel, semi-synthétique ou synthétique ou un fluide fonctionnel.

18. Composition selon la revendication 17, où la matière organique est un lubrifiant ou un fluide d'usinage de métaux ou un fluide hydraulique.

19. Utilisation de produits- selon la revendication 1 pour la stabilisation d'une matière organique sensible contre la dégradation oxydative, thermique et/ou induite par la lumière.

20. Utilisation selon la revendication 19 pour la stabilisation de polymères naturels, semi-synthétiques ou synthétiques ou de fluides fonctionnels.

21. Utilisation selon la revendication 19 pour la stabilisation de lubrifiants, de fluides d'usinage de métaux ou de fluides hydrauliques.

22. Procédé pour la stabilisation de matière organique contre la dégradation oxydative, thermique et/ou induite par la lumière, caractérisé en ce qu'on ajoute à, respectivement on applique sur cette matière au moins un produit selon la revendication 1 en tant que stabilisant.

23. Procédé selon la revendication 22 pour la stabilisation de polymères naturels, semi-synthétiques ou synthétiques ou de fluides fonctionnels.

**24.** Procédé selon la revendication 23 pour la stabilisation de polymères synthétiques ou de lubrifiants, de fluides d'usinage de métaux ou de fluides hydrauliques.

**25.** Procédé de préparation d'un mélange de substances caractérisé en ce qu'on fait réagir une oléfine de formule

$$R_1R_2C{=}CHR_3 \qquad (I)$$

sur un mélange de composés de formules II et III

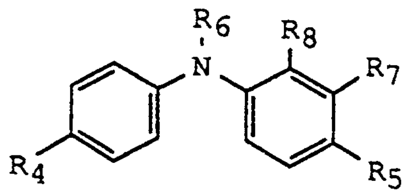
(II)

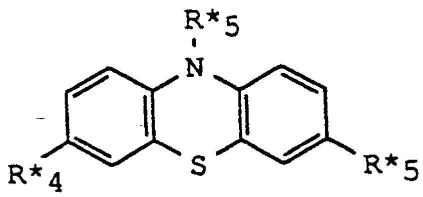
(III)

en présence d'un catalyseur acide,
où

| | |
|---|---|
| $R_1$ | représente un atome d'hydrogène, des groupes alkyle en $C_1$-$C_{25}$ ou phényle, |
| $R_2$ | représente des groupes alkyle en $C_1$-$C_{25}$, benzyle ou phényle, |
| $R_3$ | représente un atome d'hydrogène ou un groupe alkyle en $C_1$-$C_{12}$, |
| $R_4$, $R_4^*$, $R_5$ et $R_5^*$ | représentent, indépendamment les uns des autres, des atomes d'hydrogène, des groupes alkyle en $C_1$-$C_{25}$, cycloalkyle en $C_5$-$C_{12}$, benzyle, α-méthylbenzyle ou α,α-diméthylbenzyle, et |
| $R_6$ et $R_6^*$ | représentent, indépendamment l'un de l'autre, des atomes d'hydrogène, des groupes alkyle en $C_1$-$C_{18}$, alcényle en $C_3$-$C_4$, benzyle ou un reste de formule -CH($R_9$)-CH($R_{10}$)-S-$R_{11}$, ainsi que |
| $R_7$ et $R_8$ | représentent des atomes d'hydrogène ou ensemble forment un groupe bivalent |

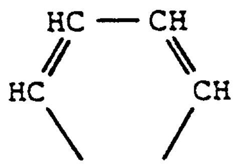

| | |
|---|---|
| $R_9$ et $R_{10}$ | représentent, indépendamment l'un de l'autre, des atomes d'hydrogène, des groupes phényle ou alkyle en $C_1$-$C_{12}$, |
| $R_{11}$ | représente des groupes alkyle en $C_4$-$C_{18}$, phényle ou $-(CH_2)_a$-CO-$OR_{12}$, |
| a | vaut 1 ou 2, et |
| $R_{12}$ | représente un groupe alkyle en $C_1$-$C_{18}$. |